(19) Europäisches Patentamt
European Patent Office
Office européen des brevets

(11) EP 3 310 799 B1

(12) EUROPEAN PATENT SPECIFICATION

(45) Date of publication and mention of the grant of the patent:
**26.08.2020 Bulletin 2020/35**

(21) Application number: **16811906.3**

(22) Date of filing: **15.06.2016**

(51) Int Cl.:
***C07H 17/04** (2006.01)* ***A61K 31/7048** (2006.01)*
***A61P 11/00** (2006.01)* ***A61P 37/00** (2006.01)*

(86) International application number:
**PCT/KR2016/006318**

(87) International publication number:
**WO 2016/204493 (22.12.2016 Gazette 2016/51)**

(54) **A NOVEL COMPOUND (KS 513) ISOLATED FROM PSEUDOLYSIMACHION ROTUNDUM VAR. SUBINTEGRUM AND THE COMPOSITION COMPRISING THE SAME AS AN ACTIVE INGREDIENT FOR PREVENTING OR TREATING ALLERGY DISEASE, INFLAMMATORY DISEASE, ASTHMA OR CHRONIC OBSTRUCTIVE PULMONARY DISEASE**

NEUARTIGE, AUS PSEUDOLYSIMACHION ROTUNDUM VAR. SUBINTEGRUM ISOLIERTE VERBINDUNG (KS 513), UND SIE ALS WIRKSTOFF ENTHALTENDE ZUSAMMENSETZUNG ZUR PRÄVENTION ODER BEHANDLUNG EINER ALLERGIEERKRANKUNG, ENTZÜNDUNGSERKRANKUNG, ASTHMA ODER CHRONISCH OBSTRUKTIVER LUNGENERKRANKUNG

NOUVEAU COMPOSÉ (KS 513) ISOLÉ DE PSEUDOLYSIMACHION ROTUNDUM VAR. SUBINTEGRUM, ET COMPOSITION LE COMPRENANT COMME INGRÉDIENT ACTIF POUR LA PRÉVENTION OU LE TRAITEMENT DE L'ALLERGIE, D'UNE MALADIE INFLAMMATOIRE, DE L'ASTHME OU D'UNE MALADIE PULMONAIRE OBSTRUCTIVE CHRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2015 KR 20150085752**

(43) Date of publication of application:
**25.04.2018 Bulletin 2018/17**

(73) Proprietors:
- **Yungjin Pharmaceutical Co., Ltd.**
  **Gangdong-gu**
  **Seoul 05336 (KR)**
- **Korea Research Institute of Bioscience and Biotechnology**
  **Daejeon 34141 (KR)**

(72) Inventors:
- **LEE, Yongnam**
  **Yongin-si**
  **Gyeonggi-do 16860 (KR)**
- **YOO, Ji-Seok**
  **Suwon-si**
  **Gyeonggi-do 16396 (KR)**
- **SHIN, Dae-Hee**
  **Seoul 06628 (KR)**
- **RYOO, Byung-Hwan**
  **Seongnam-si**
  **Gyeonggi-do 13633 (KR)**
- **OH, Sei-Ryang**
  **Daejeon 34077 (KR)**
- **AHN, Kyung-Seop**
  **Daejeon 34079 (KR)**
- **LEE, Hyeong-Kyu**
  **Daejeon 34125 (KR)**
- **LEE, Su Ui**
  **Daejeon 34082 (KR)**
- **SONG, Hyuk-Hwan**
  **Seoul 04724 (KR)**
- **RYU, Hyung Won**
  **Daejeon 35230 (KR)**

(74) Representative: **Grünecker Patent- und Rechtsanwälte**
**PartG mbB**
**Leopoldstraße 4**
**80802 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
**WO-A1-2014/104672 KR-A- 20140 087 982**
**US-A1- 2009 324 750**

- **TASKOVA R ET AL: "Iridoid glucosides of the genus Veronica s.l. and their systematic significance", PLANT SYSTEMATICS AND EVOLUTION, VIENNA, AT, vol. 231, no. 1-4, 1 January 2002 (2002-01-01), pages 1-17, XP002504848, ISSN: 0378-2697, DOI: 10.1007/S006060200008**
- **SONG, H. H. ET AL.: 'Piscroside C, a novel iridoid glycoside isolated from Pseudolysimachion rotundum var. subinegrum suppresses airway inflammation induced by cigarette smoke' JOURNAL OF ETHNOPHARMACOLOGY vol. 170, pages 20 - 27, XP029174758**
- **HAN, X. H. ET AL.: 'Two new iridoids from the stem of catalpa ovata' HELVETICA CHIMICA ACTA vol. 98, no. 3, 01 March 2015, pages 381 - 385, XP055338222**
- **JIA, Q. ET AL.: 'Pikuroside: a novel iridoid from Picrorhiza kurroa' JOURNAL OF NATURAL PRODUCTS vol. 62, no. 6, 1999, pages 901 - 903, XP002504846**

## Description

### Technical Field

The present invention relates to a novel compound (KS 513) isolated from *Pseudolysimachion* rotundum var. subintegrum, the composition comprising the compound, as well as to the compound and composition for use in a method of preventing or treating allergic disease, inflammatory disease, asthma or chronic obstructive pulmonary disease.

### Background Art

Generally, an inflammatory response is a normal response of human body associated with an edema, a pain etc in case that a tissue or a cell received any invasion causing some organic change in the tissue or cell. Recently, various kinds of cytokines have been found to be involved in the inflammatory disease.

Allergic reaction may be classified into four categories, i.e., type I, II, III and IV according to the types of response or two categories, i.e., immediate type allergic reaction such as type I, II or III, and delayed type allergic reaction such as type IV according to the types of the period from the re-sensitization time caused by allergen to the onset time of reaction.

Among them, type I allergy, being involved in IgE antibody and called as anaphylaxis type allergy, causes to a bronchial asthma, atopic diseases such as dermatitis or gastroenteritis etc, allergic rhinitis such as pollenosis, allergic conjunctivitis, food allergy and the like.

Asthma is regarded as a complex syndrome of the airways that is characterized by various clinical symptoms, for example, cough, dyspnea caused by airflow obstruction, acute or chronic airway inflammation, airway hyperresponsiveness(AHR) and structural remodeling and can be reversibly or irreversibly recoverable. Most of asthma is allergic disease and is characterized by chronic airway inflammation and bronchial hyperresponsiveness (Minoguchi K and Adachi M., Pathophysiology of asthma. In: Cherniack NS, Altose MD, Homma I. editors. Rehabilitation of the patient with respiratory disease. New York: McGraw-Hill, 1999, pp97-104).

The asthma can be classified two types, i.e., extrinsic asthma and intrinsic asthma. The extrinsic asthma is caused by exposing antigen and it is shown positive reaction in skin test or bronchial provocation test against the antigen. Usually causing ages is getting younger. It is mainly caused by House Dust Mite Dermatophagoides and pollen, epithelium of animal, fungi and so on. The intrinsic asthma is caused by upper respiratory infections, exercise, emotional instability, changing of climate of humidity and it is common to adult patient. Also, the IgE antigen of extrinsic asthma can be detected by skin test due to increasing IgE in serum.

With regards to pathophysiology, asthma is recognized by T-helper2 (Th2)-cell-driven chronic inflammation, and a variety of inflammatory mediators, such as cytokines, chemokines, signaling molecules, adhesion molecules and growth factors, from immune cells and structural cells in the airways are involved in various stages of asthma (Elias JA et al., J Clin Invest.2003, 111, 291-297). The activated inflammatory cells such as eosinophil, mast cells, alveolar macrophage etc in the bronchus of patients suffering from asthma, release various inflammatory mediators such as cystein leukotrienes, prostaglandins etc and is involved in potent bronchial constriction (Maggi E. et al., Immunotechnology1998, 3, 233-244.; Pawankar R. et al., Curr. Opin. Allergy Clin. Immunol.2001, 1, 3-6.; Barnes PJ et al., Phamacol. Rev. 1998, 50, 515-596).

Accordingly, since the reproduction of various cytokines involved in inflammatory cell activation, such as IL-4, IL-5, IL-13 etc and IgE and reproduction of cystein leukotrienes released from the inflammatory cells arc the main causes of inflammation, allergic reaction and asthma, there have been much studied to develop the inhibiting agents from the reproduction of those till now.

Generally, chronic obstructive pulmonary disease (COPD) is one of pulmonary disease caused by abnormal inflammatory disease in lung resulting in the obstruction of respiratory tract. COPD gives rise to dyspnoea resulting from the hindrance from exhausting air flow and shows different characteristics for example, the poor reversibility of an airways limitation or airways obstruction, the progressive development according to elapse time etc, from the common characteristics of asthma and may be classified into a pulmonary emphysema and chronic obstructive bronchitis (Barnes P.J., Pharmacol. Rev. 2004, 56, 515-548).

COPD has been reported as one of risk factor for cardiovascular morbidity and mortality and the fifth leading cause of death worldwide in 2001. The prevalence of chronic obstructive pulmonary disease based on Global Initiative for Chronic Obstructive Lung Disease (GOLD) criteria (a ratio of FEV1 to FVC of less than 0.7) was 17.2% (men, 25.8%; women, 9.6%) among Koreans older than 45 years (Kim, D. S. et al., Am. J. Respir. Crit. Care Med.2005, 172, 842-847.; Sin, D.D. et al., Proc. Am. Thorac. Soc.2005, 2, 8-11.; Buist A.S. et al., Lancet, 2007, 370, 741-750)

Most patients with COPD have all three pathological mechanisms (chronic obstructive bronchitis, emphysema, and mucus plugging) as all are induced by smoking, but they may differ in the proportion of emphysema and obstructive bronchitis. In developed countries, cigarette smoking is by far the most common cause of COPD, but there are several other risk factors, including air pollution (particularly, indoor air pollution from burning fuels), poor diet, and occupational

exposure. COPD is characterized by acceleration in the normal decline of lung function seen with age. The slowly progressive airflow limitation leads to disability and premature death and is quite different from the variable airway obstruction and symptoms in asthma, which rarely progresses in severity.

There have been reported that the pathophysiological action and syndrome of COPD are fundamentally different from those of asthma. Although COPD and asthma both involve inflammation in the respiratory tract, there are marked differences in the nature of the inflammatory process, with differences in inflammatory cells, mediators, response to inflammation, anatomical distribution, and response to anti-inflammatory therapy, for example, (a) in respect to inflammatory cells, mast cell, eosinophils, $D4^+$ cell (Th2), macrophages etc mainly act on the occurrence of asthma whereas neutrophils, $CD8^+$ (Tc) etc mainly act on the occurrence of COPD; (b) in respect to inflammatory mediators, leukotriens B, histamine, IL-4, IL-5, IL-13, eotaxin, RENTES, oxidative stress etc arc mainly involved in the occurrence of asthma whereas TNF-alpha, IL-8, GRO-alpha etc are mainly involved in the occurrence of COPD; (c) in respect to inflammatory syndrome, asthma shows different inflammatory syndrome by acting on the overall pulmonary tract at early age, such as AHR (airway hyperresponsiveness), epithelial shedding, fibrosis, no parenchymal involvment, muscus secretion, relatively reversible airways obstruction, cough, sneezing, dyspnea etc from that of COPD, which occurs by acting on peripheral airways at adults and shows various phenomena such as, epithelial metaplasia, parenchymal destruction, relatively irreversible airways obstruction, chronic bronchitis, emphysema etc (Barnes PJ., Chest 2000, 117, 10S-14S.; Saetta M. et al., Am. J. Respir. Crit. Care Med. 2001, 163, 1304-1309).

Histopathological studies on COPD show a predominant involvement of peripheral airways (bronchioles) and lung parenchyma, whereas asthma involves inflammation in all airways but without involvement of the lung parenchyma. There is obstruction of bronchioles, with fibrosis and infiltration with macrophages and T lymphocytes. There is destruction of lung parenchyma, as well as an increased number of macrophages and CD8 (cytotoxic) T lymphocytes (Saetta M. et al., Am. J. Respir. Crit. Care Med. 1998, 157, 822-826). Bronchial biopsies show similar changes with an infiltration of macrophages and CD8cells and an increased number of neutrophils in patients with severe COPD (Di Stefano A. et al., Am. J. Respir. Crit. Care Med. 1998, 158, 1277-1285).

In contrast to asthma, eosinophils are not prominent except during exacerbations or when patients have concomitant asthma (Fabbri L. et al., Thorax 1998, 53, 803-808.; Fabbri LM. et al., Am. J. Respir. Crit. Care Med. 2003, 167, 418-424).

Accordingly, the therapeutic approach of chronic obstructive pulmonary disease (COPD) shall be different from that of asthma; however, the present therapy has been focused on treating non-specifically both of diseases. Therefore, there have been no anti-inflammatory therapies specifically approved for COPD and the available anti-inflammatory therapies were originally developed for asthma. The challenges facing research in COPD are multi-faceted; the mechanisms underlying the complex and heterogeneous pathology of this disease require unravelling; the role of inflammation in disease progression needs to be confirmed. (Hele D. et al., Expert. Opino. Invest. Drug , 2003, 12, 5-18.; Fox, J C. et al., Curr. Opin. Pharmacol. 2009, 9, 231-242).

Improvements to the current therapy available to treat asthma in the form of longer acting beta-agonists, safer steroids and combination therapies are ongoing and for COPD anti-cholinergics provide symptomatic relief. Steroids have been used to treat exacerbations, but as yet, no treatment has been shown to impact significantly on the progressive decline in lung function in COPD or the development of asthma.

Accordingly, there have been much studied to develop new drugs with potential to successfully and specifically treat allergic disease, inflammatory disease, asthma or COPD till now.

The present inventors have been focused to develop potent treating agent derived from natural resources with safety and efficacy such as plant, animals etc having potent treating activity of allergic disease, inflammatory disease, asthma or COPD and finally, have subsequently found unexpectedly surprising results, for example, potent anti-inflammatory, anti-allergy and anti-asthma activity of the extract of *Pseudolysimachion* longifolium (Korean Patent No. 10-860080 B1 and US 2012/0183632 A1) and various compounds isolated therefrom such as, verproside (6-*O*-3,4-dihydroxybenzoyl catalpol), picroside II (6-*O*-4-hydroxy-3-methoxybenzoyl catalpol), verminoside (6-*O*-3,4-Dihydroxy cinnamoyl catalpol), 6-*O*-veratroyl catalpol (6-*O*-3,4-Dimethoxy benzoyl catalpol), minecoside (6-*O*-3-hydroxy-4-methoxycinnamoyl catalpol), catalpol and the like (Korean Patent Publication No. 10-2006-125499 A1); potent anti-inflammatory, anti-allergy and anti-asthma activity of the novel purified extract containing abundant active ingredients such as catalpol derivatives from the extract of *Pseudolysimcichion* rotundum var subintegrum (ATC2: Korean Patent No. 1476045 B1, ATC1: Korean Patent No. 1504651 B1), WO2011/101672 A1 anti-COPD activity of those extract (Korean Patent No. 1476095 B1) and novel compound (KS-534) isolated therefrom till now.

US 2009/324750 discloses a composition comprising an extract of Pseudolysimachion genus plant, and the catalpol derivatives isolated therefrom having anti-inflammatory, antiallergic and anti-asthmatic activity.

Song, H. H. et al., "Piscroside C, a novel iridoid glycoside isolated from Pseudolysimachion rotundum var. subinegrum suppresses airway inflammation induced by cigarette smoke", Journal of Ethnopharmacology, vol. 170, pages 20 - 27, disclose the protective effects of the iridoid glycoside, piscroside C isolated from P. rotundum var. subintegrum against inflammatory responses.

Taskova R. et al., "Iridoid glucosides of the genus Veronica s.l. and their systematic significance", Plant Systematics and Evolution, Vienna, AT, vol. 231, no. 1-4, pages 1 - 17, disclose iridoid glucosides that were isolated from Veronica L.

Jia, Q. et al., "Pikuroside: a novel iridoid from Picrorhiza kurroa", Journal of Natural Products, vol. 62, no. 6, pages 901 - 903, disclose an iridoid, pikuroside, which was isolated from the roots of Picrorhizakurroa.

*Pseudolysimachion* rotundum var subintegrum, is a perennial herb distributed in Korea, China, Japan, Ostrov Sakhalin, and Russia.

Based on the previous studies on the anti-inflammatory, anti-allergy and anti-asthma activity of the extract of *Pseudolysimcichion* rotundum var *subintegrum,* the present inventors have tried to develop novel compound showing anti-inflammatory, anti-allergy, anti-asthma and anti-COPD activity isolated from the extract of *Pseudolysimcichion rotundum* var *subintegrum.*

However, there has been not reported or disclosed on novel compound (KS-513) showing anti-inflammatory, anti-allergy, anti-asthma and anti-COPD activity isolated from the extract of *Pseudolysimachion* rotundum var *subintegrum* in the above cited literatures,

Accordingly, the present inventors have found novel compound showing anti-inflammatory, anti-allergy, anti-asthma and anti-COPD activity isolated from the extract of *Pseudolysimcichion* rotundum var subintegrum and the inventive compound showed potent anti-inflammatory, anti-allergy, anti-asthma and anti-COPD activity through various tests, for example, (1) cyto-toxicity test using by RAW264.7 cell line, (2) an effect on the mRNA expression of pro-inflammatory medicated enzyme such as iNOS or COX-2, and pro-inflammatory medicated cytokines such as IL-lbeta, IL-6, TNF-alpha in RAW264.7 cell line induced by LPS, (3) an inhibition effect on iNOS protein expression involved in NO reproduction and the level of NO reproduction, (4) an inhibition effect on the COX-2 protein expression and the reproduced level of PGE2, (5) an inhibition effect on the produce level of IL-lbeta, IL-6, TNF-alpha etc.

**Disclosure of Invention**

The invention is defined in the appended claims.

The present invention provides a novel compound (KS513) isolated from the extract of *Pseudolysimcichion* rotundum var subintegrum.

The present invention also provides a pharmaceutical composition and a health food comprising the novel compound (KS513) isolated from the extract of *Pseu-dolysimachion* rotundum var subintegrum as an active ingredient in an effective amount to treat and prevent allergic disease, inflammatory disease, asthma or COPD disease.

More specifically, the invention provides (1aS,1bS,2S,4S,5aR,6S,6aS)-1a-(hydroxymethyl)-4-methoxy-2-(((2S,3R,4S,5S,6R)-3,4,5-trihydroxy-6-(hydroxymethyl)tetrahydro-2H-pyran-2-yl)oxy)octahydrooxireno[2',3':4,5]cyclopenta[1,2-c]pyran-6-yl4-hydroxybenzoate(KS-513) represented by the following chemical formula (1), a pharmaceutically acceptable salt or solvates thereof:

[Chemical Formula 1]

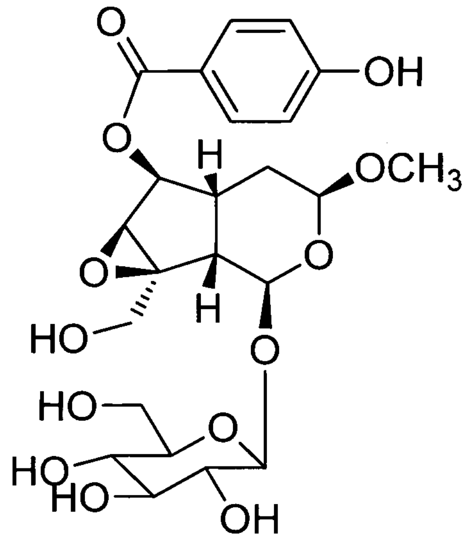

(1).

The invention further provides a pharmaceutical composition comprising the compound (KS513), the pharmaceutically acceptable salt or solvates thereof as set forth above.

The invention further provides the compound (KS513), or the pharmaceutically acceptable salt or solvates thereof as set forth above, or the pharmaceutical composition as set forth above for use in a method of treating or preventing allergic disease, inflammatory disease, asthma or chronic obstructive pulmonary disease (COPD) in mammals, wherein the method comprises administering a therapeutically effective amount of said compound (KS513), said pharmaceutically acceptable salt or solvates thereof or of said pharmaceutical composition into the mammal suffering from allergic disease, inflammatory disease, asthma or chronic obstructive pulmonary disease (COPD).

The invention further provides a health functional food comprising a therapeutically effective amount of the compound (KS513), the pharmaceutically acceptable salt or solvates thereof as set forth above as an active ingredient.

The invention further provides a health care food comprising a therapeutically effective amount of the compound (KS513), the pharmaceutically acceptable salt or solvates thereof as set forth above, together with a sitologically acceptable additive.

The invention further provides the health functional food as set forth above for use in a method of preventing or alleviating allergic disease, inflammatory disease, asthma or chronic obstructive pulmonary disease (COPD).

The invention further provides the health care food as set forth above for use in a method of preventing or alleviating allergic disease, inflammatory disease, asthma or chronic obstructive pulmonary disease (COPD).

The present invention also provides the novel compound (KS513) isolated from the extract of *Pseudolysimachion* rotundum var subintegrum for use in a method of treating or preventing inflammatory disease, allergic disease, asthma or COPD.

Further disclosed is a method of treating or preventing inflammatory disease, allergic disease, asthma or COPD in a mammal or human comprising administering to said mammal or human an effective amount of the novel compound (KS513) isolated from the extract of *Pseudolysimcichion* rotundum var subintegrum, together with a pharmaceutically acceptable carrier thereof.

The inventive novel compound can be transformed into their pharmaceutically acceptable salt and solvates by the conventional method well known in the art. For the salts, acid-addition salt thereof formed by a pharmaceutically acceptable free acid thereof is useful and can be prepared by the conventional method. For example, after dissolving the compound in the excess amount of acid solution, the salts are precipitated by the water-miscible organic solvent such as methanol, ethanol, acetone or acetonitrile to prepare acid addition salt thereof and further the mixture of equivalent amount of compound and diluted acid with water or alcohol such as glycol monomethylether, can be heated and subsequently dried by evaporation or filtrated under reduced pressure to obtain dried salt form thereof.

As a free acid of above-described method, organic acid or inorganic acid can be used. For example, organic acid such as methansulfonic acid, p-toluensulfonic acid, acetic acid, trifluoroacetic acid, citric acid, maleic acid, succinic acid, oxalic acid, benzoic acid, lactic acid, glycolic acid, gluconic acid, galacturonic acid, glutamic acid, glutaric acid,

glucuronic acid, aspartic acid, ascorbic acid, carbonylic acid, vanillic acid, hydroiodic acid and the like, and inorganic acid such as hydrochloric acid, phosphoric acid, sulfuric acid, nitric acid, tartaric acid and the like can be used herein.

Further, the pharmaceutically acceptable metal salt form of inventive compounds may be prepared by using base. The alkali metal or alkali-earth metal salt thereof can be prepared by the conventional method, for example, after dissolving the compound in the excess amount of alkali metal hydroxide or alkali-earth metal hydroxide solution, the insoluble salts are filtered and remaining filtrate is subjected to evaporation and drying to obtain the metal salt thereof. As a metal salt of the present invention, sodium, potassium or calcium salt are pharmaceutically suitable and the corresponding silver salt can be prepared by reacting alkali metal salt or alkali-earth metal salt with suitable silver salt such as silver nitrate.

The pharmaceutically acceptable salt of the compound comprise all the acidic or basic salt which may be present at the compounds, if it does not indicated specifically herein. For example, the pharmaceutically acceptable salt of the present invention comprise the salt of hydroxyl group such as the sodium, calcium and potassium salt thereof; the salt of amino group such as the hydrogen bromide salt, sulfuric acid salt, hydrogen sulfuric acid salt, phosphate salt, hydrogen phosphate salt, dihydrophosphate salt, acetate salt, succinate salt, citrate salt, tartarate salt, lactate salt, mandelate salt, methanesulfonate (mesylate) salt and p-toluenesulfonate (tosylate) salt etc, which can be prepared by the conventional method well known in the art.

There may exist in the form of optically different diastereomers since the compounds have unsymmetrical centers, accordingly, the compounds of the present invention comprise all the optically active isomers, R or S stereoisomers and the mixtures thereof. Present invention also comprises all the uses of racemic mixture, more than one optically active isomer or the mixtures thereof as well as all the preparation or isolation method of the diastereomer well known in the art.

The compounds of the invention may be isolated from the isolation or purification method which is well known in the art such as Silica gel column chromatography or re-crystalization method, for example, the method disclosed in Korea Patent Publication No. 10-2006-125499; or chemically synthesized by the well-known methods in the art, which are merely exemplary and in no way limit the invention.

The inventive novel compound (KS 513) may be prepared from *Pseudolysimachion rotundum* var *subintegrum,* by following procedure:

For example, novel compound (KS 513) is characterized by being prepared by the process of; adding at least one extracting solvent selected from water, C1-C4 lower alcohol such as methanol, ethanol, butanol etc or the mixtures thereof, preferably, mixture of water and methanol, more preferably, 10 - 100 % (w/w) methanol in water to dried *Pseudolysimachion* rotundum var *subintegrum* at the 1st step; subjecting to at least one extraction method selected from reflux extraction with hot water, cold water extraction, ultra-sonication or conventional extraction, preferably cold water extraction at the temperature ranging from 10 to 150°C, preferably from 20 to 70°C, for the period ranging from 30 mins to 72 hours, preferably, 1 to 48 hours, more preferably, cold water extraction at the temperature ranging from 10 to 60°C, preferably from 20 to 50°C, for the period ranging from 30 mins to 72 hours, preferably, 6 to 48 hours repeatedly, and then reflux extraction at the temperature ranging from 40 to 120°C, preferably from 60 to 90°C, for the period ranging from 30 mins to 72 hours, preferably, 6 to 48 hours, repeatedly, to afford the 1st extract at 2nd step; subjecting to filtration, concentration under vaccuo and drying method to afford dried crude extract at the 3rd step; and subjecting to at least one purification process selected from (i) reverse phase partition chromatography, (ii) flash column chromatography, (iii) RP C18 column chromatography, (iv) Silica gel column chromatography, (v) ion exchange chromatography or (iv) size exclusion chromatography repeatedly to afford novel compound (KS 513) of the present invention.

The term "pharmaceutically acceptable carriers or excipients" defined herein comprises "pharmaceutical additives, the inactive ingredients used to make up a medication. They include dyes, flavors, binders, emollients, fillers, lubricants, preservatives, and many more classifications. Common excipients include cornstarch, lactose, talc, magnesium stearate, sucrose, gelatin, calcium stearate, silicon dioxide, shellac and glaze, which has been well-known in the art (*See,* Home-page of Food and Drug Administration: www.fda.gov or drug information online: www.drugs.com) or previous literature (for example, Rowe, Raymond C et al., Handbook of Pharmaceutical Excipients, Pharmaceutical Press, 7th Edition, 2012)

The term "prevent" disclosed herein comprises any act to inhibit or postpone the occurrence of certain disease or disorder disclosed herein by way of administrating the inventive composition; and the term "treat" disclosed herein comprises any act to alleviate or favorably changing the symptom associated with certain disease or disorder disclosed herein by way of administrating the inventive composition.

The present inventors have found that the novel compound (KS 513) may be prepared from *Pseudolysimachion* rotundum var subintegrum showed potent anti-inflammatory, anti-allergy, anti-asthma and anti-COPD activity through various tests, (1) cyto-toxicity test using by RAW264.7 cell line, (2) an effect on the mRNA expression of pro-inflammatory medicated enzyme such as iNOS or COX-2, and pro-inflammatory medicated cytokines such as IL-1beta, IL-6, TNF-alpha in RAW264.7 cell line induced by LPS, (3) an inhibition effect on iNOS protein expression involved in NO reproduction and the level of NO reproduction, (4) an inhibition effect on the COX-2 protein expression and the reproduced

level of PGE2, (5) an inhibition effect on the produced level of IL-lbeta, IL-6, TNF-alpha etc.

Accordingly, in accordance with the other aspect of the present invention, present invention provide a pharmaceutical composition or a health functional food comprising the novel compound (KS513) isolated from the extract of *Pseudolysimachion* rotundum var subintegrum as an active ingredient in an effective amount to treat and prevent allergic disease, inflammatory disease, asthma or COPD disease.

Present invention provide a pharmaceutical composition or a health functional food comprising the novel compound (KS513) isolated from the extract of *Pseudolysimachion* rotundum var subintegrum and the pharmaceutically acceptable carriers or excipients, for the treatment or prevention of allergic disease, inflammatory disease, asthma or chronic obstructive pulmonary disease (COPD).

In accordance with another aspect of the present invention, there is also provided the novel compound (KS513) isolated from the extract of *Pseudolysimachion* rotundum var subintegrum for use in a method of treating or preventing allergic disease, inflammatory disease, asthma or chronic obstructive pulmonary disease (COPD).

In accordance with another aspect of the present invention, there is also provided the novel compound (KS513) isolated from the extract of *Pseudolysimachion* rotundum var subintegrum and the pharmaceutically acceptable carriers or excipients for use in a method of treating or preventing allergic disease, inflammatory disease, asthma or chronic obstructive pulmonary disease (COPD).

In accordance with another aspect of the disclosure there is also disclosure method of treating or preventing allergic disease, inflammatory disease, asthma or chronic obstructive pulmonary disease (COPD) in mammal, wherein the method comprises administering a therapeutically effective amount of the novel compound (KS513) isolated from the extract of *Pseudolysimachion* rotundum var subintegrum into the mammal suffering from allergic disease, inflammatory disease, asthma or chronic obstructive pulmonary disease (COPD).

In accordance with another aspect of the disclosure, there is also disclosed a method of treating or preventing allergic disease, inflammatory disease, asthma or chronic obstructive pulmonary disease (COPD) in mammals, wherein the method comprises administering a composition comprising therapeutically effective amount of the novel compound (KS513) isolated from the extract of *Pseudolysimachion* rotundum var subintegrum and the pharmaceutically acceptable carriers or excipients, into the mammal suffering from allergic disease, inflammatory disease, asthma or chronic obstructive pulmonary disease (COPD).

The inventive composition for treating and preventing allergic disease, inflammatory disease, asthma or chronic obstructive pulmonary disease (COPD) may comprise the above compounds as 0.1 ~ 99%, preferably, 0.1 ~ 50% by weight based on the total weight of the composition.

The composition according to the present invention can be provided as a pharmaceutical composition containing pharmaceutically acceptable carriers, adjuvants or diluents, e.g., lactose, dextrose, sucrose, sorbitol, mannitol, xylitol, erythritol, maltitol, starches, acacia rubber, alginate, gelatin, calcium phosphate, calcium silicate, cellulose, methyl cellulose, polyvinyl pyrrolidone, water, methylhydroxy benzoate, propylhydroxy benzoate, talc, magnesium stearate and mineral oil. The formulations may additionally include fillers, anti-agglutinating agents, lubricating agents, wetting agents, flavoring agents, emulsifiers, preservatives and the like. The compositions of the invention may be formulated so as to provide quick, sustained or delayed release of the active ingredient after their administration to a patient by employing any of the procedures well known in the art.

For example, the compositions of the present invention can be dissolved in oils, propylene glycol or other solvents that are commonly used to produce an injection. Suitable examples of the carriers include physiological saline, polyethylene glycol, ethanol, vegetable oils, isopropyl myristate, etc., but are not limited to them. For topical administration, the extract of the present invention can be formulated in the form of ointments and creams.

Pharmaceutical formulations containing present composition may be prepared in any form, such as oral dosage form (powder, tablet, capsule, soft capsule, aqueous medicine, syrup, elixirs pill, powder, sachet, granule), or topical preparation (cream, ointment, lotion, gel, balm, patch, paste, spray solution, aerosol and the like), or injectable preparation (solution, suspension, emulsion).

The composition of the present invention in pharmaceutical dosage forms may be used in the form of their pharmaceutically acceptable salts, and also may be used alone or in appropriate association, as well as in combination with other pharmaceutically active compounds.

The desirable dose of the inventive extract or compound varies depending on the condition and the weight of the subject, severity, drug form, route and period of administration, and may be chosen by those skilled in the art. However, in order to obtain desirable effects, it is generally recommended to administer at the amount ranging from 0.0001 to 1000mg/kg, preferably, 0.001 to 100mg/kg by weight/day of the inventive extract of the present invention. The dose may be administered in single or divided into several times per day.

The pharmaceutical composition of present invention can be administered to a subject animal such as mammals (rat, mouse, domestic animals or human) *via* various routes. All modes of administration are contemplated, for example, administration can be made orally, rectally or by intravenous, intramuscular, subcutaneous, intracutaneous, intrathecal, epidural or intracerebroventricular injection.

The present invention also provides a pharmaceutical composition and a health food comprising the novel compound (KS513) isolated from the extract of *Pseudolysimcichion* rotundum var subintegrum as an active ingredient in an effective amount to treat and prevent allergic disease, inflammatory disease, asthma or COPD disease.

The present invention also provides the novel compound (KS513) isolated from the extract of *Pseudolysimachion* rotundum var subintegrum for use in a method of treating or preventing inflammatory disease, allergic disease, asthma or COPD.

Further disclosed is a method of treating or preventing inflammatory disease, allergic disease, asthma or COPD in a mammal or human comprising administering to said mammal or human an effective amount of the novel compound (KS513) isolated from the extract of *Pseudolysimachion* rotundum var subintegrum, together with a pharmaceutically acceptable carrier thereof.

The inventive extract of the present invention also can be used as a main component or additive and aiding agent in the preparation of various functional health food and health care food.

Accordingly, it is the other object of the present invention to provide a health functional food comprising a therapeutically effective amount of the novel compound (KS513) isolated from the extract of *Pseudolysimachion* rotundum var subintegrum containing active ingredients for the prevention or alleviation of allergic disease, inflammatory disease, asthma or chronic obstructive pulmonary disease (COPD).

The term "a functional health food" defined herein" the functional food having enhanced functionality such as physical functionality or physiological functionality by adding the extract of the present invention to conventional food to prevent or improve the purposed diseases in human or mammal.

It is the other object of the present invention to provide a health care food comprising a therapeutically effective amount of the novel compound (KS513) isolated from the extract of *Pseudolysimachion* rotundum var subintegrum, together with a sitologically acceptable additive for the prevention or alleviation of allergic disease, inflammatory disease, asthma or chronic obstructive pulmonary disease (COPD).

The term "a health care food" defined herein "the food containing the extract or compound(s) of the present invention showing no specific intended effect but general intended effect in a small amount of quantity as a form of additive or in a whole amount of quantity as a form of powder, granule, capsule, pill, tablet etc.

The term "a sitologically acceptable additive" defined herein comprises "any substance the intended use which results or may reasonably be expected to result-directly or indirectly-in its becoming a component or otherwise affecting the characteristics of any food", and can be classified into three groups according to its origin, i.e., (1) chemically synthetic additive such as ketones, glycin, potassium citrate, nicotinic acid, etc; (2) natural additive such as persimmon dye, licorice extract, crystalline cellulose, gua dum etc; (3) the mixed additive therewith such as sodium L-glutamate, presevatives, tar dye etc, or various categories according to its function in the food, for example, thickening agent, maturing agent, bleaching agent, sequestrant, humectant, anti-caking agent, clarifying agents, curing agent, emulsifier, stabilizer, thickener, bases and acid, foaming agents, nutrients, coloring agent, flavoring agent, sweetner, preservative agent, antioxidant, etc, which has been well-known in the art or previous literature (*See,* "Codex General Standard for Food Additives" (GSFA, Codex STAN 192-1995) in Home-page of GSFA Online: www.codexalimentarius.net/gsfaonline/index.html).

If a substance is added to a food for a specific purpose in that food, it is referred to as a direct additive and indirect food additives are those that become part of the food in trace amounts due to its packaging, storage or other handling.

The term "health care foods or health functional foods" disclosed herein can be contained in food, health beverage, dietary supplement etc, and may be formulated into a form of pharmaceutically dosing form such as a powder, granule, tablet, suspension, emulsion, syrup, chewing tablet, capsule, beverage etc; or the food form, for example, bread, rice cake, dry fruit, candy, chocolate, chewing gum, ice cream, milk such as low-fat milk, lactose-hydrolyzed milk, goat-milk, processed milk, milk product such as fermented milk, butter, concentrated milk, milk cream, butter oil, natural cheese, processed cheese, dry milk, milk serum etc, processed meat product such as hamburger, ham, sausage, bacon etc, processed egg product, fish meat product such as fish cake etc, noodle products such as instant noodles, dried noodles, wet noodles, fried noddles, non-fried noodles, gelatinized dry noodles, cooked noodles, frozen noodles, Pasta etc, tea product such as tea bag, leached tea etc, health drinks such as fruit drinks, vegetable drinks, carbonated soft drinks, soymilk drinks, lactic beverage mixed beverage, etc, seasoning food such as soy sauce, soybean paste, red pepper paste, chunjang (a kind of fermented soybean product colored by caramel), cheonggukjang (natural fermented soybean by B. subtillis), mixed paste, vinegar, sauce, ketchup, curry, dressing etc, margarine, shortening, pizza etc, but not intended herein to limit thereto, for preventing or improving of purposed disease.

Also, above described extract or compound can be added to food or beverage for prevention and improvement of purposed disorder. The amount of above described extract or a compound(s) in food or beverage as a functional health food or health care food may generally range from about 0.01 to 100 w/w % of total weight of food for functional health food composition. In particular, although the preferable amount of the extract of the present invention in the functional health food, health care food or special nutrient food may be varied in accordance to the intended purpose of

each food, it is preferably used in general to use as an additive in the amount of the extract or a compound(s) of the present invention ranging from about 0.01 to 5% in food such as noodles and the like, from 40 to 100% in health care food on the ratio of 100% of the food composition.

Providing that the health beverage composition of present invention contains above described extract or a compound(s) as an essential component in the indicated ratio, there is no particular limitation on the other liquid component, wherein the other component can be various deodorant or natural carbohydrate etc such as conventional beverage. Examples of aforementioned natural carbohydrate are monosaccharide such as glucose, fructose etc; disaccharide such as maltose, sucrose etc; conventional sugar such as dextrin, cyclodextrin; and sugar alcohol such as xylitol, and erythritol etc. As the other deodorant than aforementioned ones, natural deodorant such as taumatin, stevia extract such as levaudiosideA, glycyrrhizin *et al.,* and synthetic deodorant such as saccharin, aspartam *et al.,* may be useful favorably. The amount of above described natural carbohydrate is generally ranges from about 1 to 20 g, preferably 5 to 12 g in the ratio of 100 mℓ of present beverage composition.

The other components than aforementioned composition are various nutrients, a vitamin, a mineral or an electrolyte, synthetic flavoring agent, a coloring agent and improving agent in case of cheese, chocolate *et al.,* pectic acid and the salt thereof, alginic acid and the salt thereof, organic acid, protective colloidal adhesive, pH controlling agent, stabilizer, a preservative, glycerin, alcohol, carbonizing agent used in carbonate beverage *et al.* The other component than aforementioned ones may be fruit juice for preparing natural fruit juice, fruit juice beverage and vegetable beverage, wherein the component can be used independently or in combination. The ratio of the components is not so important but is generally range from about 0 to 20 (w/w) % per 100 (w/w) % present composition. Examples of addable food comprising aforementioned extract or compound therein are various food, beverage, gum, vitamin complex, health improving food and the like.

Inventive compound(s) of the present invention has no toxicity and adverse effect therefore; they can be used with safe.

It will be apparent to those skilled in the art that various modifications and variations can be made in the compositions, use and preparations of the present invention without departing from the scope of the invention as defined by the appended claims.

The present invention is more specifically explained by the following examples. However, it should be understood that the present invention is not limited to these examples in any manner.

## Advantageous Effects of Invention

As described in the present invention, inventive the novel compound (KS513) isolated from the extract of *Pseuclolysimachion* rotundum var subintegrum*subintegrum* showed potent anti-inflammatory, anti-allergy, anti-asthma and anti-COPD activity through various tests, (1) cyto-toxicity test using by RAW264.7 cell line, (2) an effect on the mRNA expression of pro-inflammatory medicated enzyme such as iNOS or COX-2, and pro-inflammatory medicated cytokines such as IL-lbeta, IL-6, TNF-alpha in RAW264.7 cell line induced by LPS, (3) an inhibition effect on iNOS protein expression involved in NO reproduction and the level of NO reproduction, (4) an inhibition effect on the COX-2 protein expression and the reproduced level of PGE2, (5) an inhibition effect on the produced level of IL-lbeta, IL-6, TNF-alpha etc.

## Brief Description of Drawings

The above and other objects, features and other advantages of the present invention will more clearly understood from the following detailed description taken in conjunction with the accompanying drawings, in which;

Fig. 1 shows the effect of test sample on the cyto-toxicity in RAW264.7 cell line;

Fig. 2 shows the effect of test sample on the mRNA expression of pro-inflammatory medicated enzyme such as iNOS or COX-2, and pro-inflammatory medicated cytokines such as IL-lbeta, IL-6, TNF-alpha in RAW264.7 cell line;

Fig. 3 shows the inhibition effect of test sample on iNOS protein expression involved in NO reproduction and the level of NO reproduction using by Western blot assay and Nitric Oxide assay (Statistical analysis was performed by Student's-t-test and P value (**, <0.05) was regarded as being statistical significant);

Fig. 4 presents the inhibition effect of test sample on the COX-2 protein expression and the reproduced level of PGE2 using by Western blot assay and enzyme immunoassay (EIA) method (Statistical analysis was performed by Student's-t-test and P value (**, <0.01 and ***, <0.001) was regarded as being statistical significant);

Fig. 5 presents the effect of the inventive compound on the released level of IL-1beta, IL-6, TNF-alpha etc (Statistical analysis was performed by Student's-t-test and P value (*, <0.05, (**, <0.01 and ***, <0.001) was regarded as being statistical significant)

It will be apparent to those skilled in the art that various modifications and variations can be made in the

compositions, use and preparations of the present invention without departing from the scope of the invention as defined by the appended claims.

The present invention is more specifically explained by the following examples. However, it should be understood that the present invention is not limited to these examples in any manner.

## EXAMPLES

The following Reference Example, Examples and Experimental Examples are intended to further illustrate the present invention without limiting its scope.

### Reference Example 1. Analysis Apparatus

Melting point was determined with no correction by melting point determination apparatus (Koflermicrohostage); optical rotation by Jasco P-1020 polarimeter; UV data by UV-VIS 2450 spectrometer; FT-IR spectra by Jasco FT/IR-4200; NMR spectra by Varian UNITY 400 MHz FT-NMR spectrometer using by TMS as an internal standard; HRESIMS by Waters Q-TOF Premier spectrometer; and HPLC analysis by Gilson HPLC using by UV/VIS-155 detector and pump 305 in the experiment.

### Example 1. Preparation of novel compound (KS 513) from *Pseudolysimachion rotundum* var *subintegrum*

1-1. Preparation of crude extract

4.0 kg of dried *Pseudolysimachion* rotundum var subintegrum (cultivated at 244, Soimyeon Eumseong-gun Chungcheongbuk-do in Korea according to GAP, KRIBB 0020697, plant extract bank of KRIBB, Taejeon, KOREA) cut into small pieces and mixed with 10L of methanol. The mixture was stirred at room temperature for 24 hours and extracted with reflux extraction at 78°C for 12 hours to collect the filtrate, two times. The extract was filtered with filter paper to remove the debris. The collected filtrate was concentrated by rotary evaporator (EYELA, N-2100, Japan) at 55~65°C under reduced pressure and dried with freezing dryer to obtain 397.4g of dried crude extract

1-2. Preparation of purified extract

200 g of dried crude extract was dissolved in mixture solvent (25% MeOH-water) and subjected to further purification using by preparative reverse phase chromatography (Zeoprep C18, 75 μm, 200 x 250 mm, Zeochem, Louisville, U. S. A). The eluting fractions (f1 - f4) were collected and concentrated under vaccuo. 5.0 g of the fraction f2 was loaded to medium pressure liquid chromatography (MPLC) using by column: RP C-18 (Zeoprep C18, 20 x 250 mm, 10 μm, Zeochem, Louisville, U. S. A.) and eluting solvent (MeOH-water solution = 2:8, 3:7, 4:6, 10:0) to afford 5 sub-fractions, i.e., f2a, f2b, f2c, f2d and f2e.

1-3. Preparation of novel compound KS513

2.3 g of sub-fraction (f2b) was subjected to semi-preparative HPLC (Synergy Polar-RP 4 μm, 21.2 x 250 mm, Phenomenex, Torrance, CA, U.S.A., 23% MeCN in $H_2O$) to obtain white amorphous powdered novel iridoid compound (1aS,1bS,2S,4S,5aR,6S,6aS)-1a-(hydroxymethyl)-4-methoxy-2-(((2S,3R,5S,6R)-3,4,5-trihydroxy-6-(hydroxymethyl)tetrahydro-2H-pyran-2-yl)oxy)octahydrooxireno[2',3':4, 5]cyclopenta[1,2-c]pyran-6-yl 4-hydroxybenzoate} (KS-513; $C_{23}H_{29}O_{13}$) showing following physico-chemical properties:

$[\alpha]^{20}_D$ -64.0° (c 0.2, MeOH).
HRESIMS (observed m/z 513.1609 [M-H]-): quasimolecular 3:1 ion cluster
IR spectrum: 3412 $cm^{-1}$ (hydroxyl group); 1692 $cm^{-1}$. (α,β-unsaturated ester carbonyl)
$^1H$ and $^{13}C$ NMR spectra: Table 1

[Table 1]

| 1H (400 MHz) and 13C (100 MHz) NMR spectroscopic data for KS-513 in DMSO-d6 | | |
|---|---|---|
| Position | KS-513 | |
| | $\delta_C$ | $\delta_H$ (*J* in Hz) |
| Agluc | | |

(continued)

| 1H (400 MHz) and 13C (100 MHz) NMR spectroscopic data for KS-513 in DMSO-d6 | | |
|---|---|---|
| Position | KS-513 | |
| | $\delta_C$ | $\delta_H$ (*J* in Hz) |
| 1 | 93.2 | 5.10, d (7.6) |
| 3 | 98.0 | 4.68, dd (9.2, 2.4) |
| 4 | 28.2 | 1.72, d (13.6) |
| | | 1.56, m |
| 5 | 34.4 | 2.33, m |
| 6 | 76.0 | 5.24, d (9.0) |
| 7 | 57.9 | 3.68, d (9.08) |
| 8 | 65.5 | |
| 9 | 41.5 | 2.29, t (8.0) |
| 10 | 58.3 | 3.85, m |
| | | 3.85, m |
| 11 | 58.3 | 3.37, s |
| Aroyl | | |
| 1' | 119.8 | |
| 2' | 131.6 | 7.84, d (8.4) |
| 3' | 115.4 | 6.87, d (8.4) |
| 4' | 162.8 | |
| 5' | 115.4 | 6.87, d (8.4) |
| 6' | 131.6 | 7.84, d (8.4) |
| 7" | 165.8 | |
| Glc | | |
| 1" | 97.4 | 4.59, d (8.0) |
| 2" | 73.4 | 3.04, m |
| 3" | 76.6 | 3.19, m |
| 4" | 70.3 | 3.04, m |
| 5" | 77.4 | 3.13, m |
| 6" | 61.3 | 3.69, d (8.8) |
| | | 3.43, dd (12.0, 5.6) |

**Experimental Example 1. Cytotoxicity test.**

In order to determine the cytotoxicity of inventive compound, following cytotoxicity test using by RAW264.7 cell line, was performed by the method disclosed in the literature (Lee, S. U., et al., 2010, Anti-resorptive saurolactam exhibits in vitro anti-inflammatory activity via ERK-NK-kappa B signaling pathway, International Immunopharmacology, 10, pp298-303).

1-1.Evaluation of cytotoxicity in RAW264.7 cell

RAW264.7 cell (TIB-71, ATCC), a mouse macrophage cell, was suspended in 5% FBS (Fetal Bovine Serum)-supplemented DMEM (Dulbecco's Modified Eagle Medium, Gibco) medium in a concentration of 1 x $10^5$ cells/ml, and 100 μL of the suspension was inoculated into 96 well plates to adhere cell to the plate for 4 hours. Various concentrations of test sample (KS 513 compound) were treated therewith and cultured for 24 hours. 10 μL of CCK-8 solution was mixed therewith according to the manufacture's manual kit (Dojindo Co. Ltd), reacted from 30 mins to 4 hours, and the absorbance of the solution was determined at 570 nm. The cell viability was calculated according to following Math formulae 1 based on negative control group treated with 0.2% DMSO and the result was shown in Table 2.

[Math formulae 1]

Cell viability (%) = OD 570nm (test group)/ OD 570nm (negative control group) x 100

At the result, as shown in Fig. 1 and Table 2, it has been confirmed that KS 513 compound showed more than 95% cell viability at less than 80 μM and therefore, it did not show cytotoxicity in RAW264.7 cell.

[Table 2]

| Summary of cell viability by KS-513 in RAW264.7 cells | | |
|---|---|---|
| Test sample | concentration(μM) | RAW264.7 cell viability(%, mean±variation) |
| Negative control group | 0 | 100.00 ± 4.17 |
| KS-513 | 2.5 | 99.58 ± 3.66 |
| | 5 | 99.26 ± 5.68 |
| | 10 | 98.18 ± 3.28 |
| | 20 | 94.66 ± 3.03 |
| | 40 | 97.23 ± 7.00 |
| | 80 | 95.14 ± 2.73 |

**Experimental Example 2. Inhibition on the** mRNA expression of pro-inflammatory medicated enzyme and pro-inflammatory medicated cytokines.

In order to determine the inhibitory effect on the mRNA expression of pro-inflammatory medicated enzyme such as iNOS or COX-2, and pro-inflammatory medicated cytokines such as IL-lbeta, IL-6, TNF-alpha in RAW264.7 cell line induced by LPS, following test using by real-time PCR (real-time polymerase chain reaction, quantitative real time polymerase chain reaction, qPCR) was performed according to the method disclosed in the literature (Livak, K.J., Schimittgen T.D., 2001, Analysis of relative gene expression data using real-time quantitiative PCR and the 2 (-Delta Delta C(T) method, Methods25, p402-408):

2-1. Procedure

RAW264.7 cell was inoculated into 6-well-plates in the concentration of 1 x $10^6$ cell/ well to incubate for 12 hours. Various concentration of KS-513 compound (2.5, 5, 10 and 20 μM) was pre-treated therewith and 1μg/mL of LPS (L6529, Sigma) was added thereto to incubate for 12 hours.

To extract total RNA, RNA was extracted using by Triazol B (invitrogen) and cDNA was synthesized using by Omniscript RT kit (205113, Qiagen, Gmbh, Hilden, Germany) after the quantification. The synthesized cDNA was mixed with its template and the primers shown in Table 3 (SEQ.I.D. 1 - 14), performed to denaturation at 94°C for 5 mins using by PCR mix (PCR master Mix, Bioneer, Korea), and reacted as follows: 1cycle for pre-denaturation; at 95°C for 30 sec, 60°C for 45 sec, 72°C for 45 sec, 30 cycles for denaturation; 10 mins at 72°C, lcycle for final extension. (Fig. 2)

[Table 3]

| Primer sequence used in this study | | | |
|---|---|---|---|
| Gene | primer | | SEQ.I.D. |
| iNOS | sense | 5'-CCTTGTTCAGCTACGCCTTC-3' | 1 |
| | antisense | 5'-AAGGCCAAACACAGCATACC-3' | 2 |
| COX-1 | sense | 5'-GTGGCTATTTCCTGCAGCTC-3' | 3 |
| | antisense | 5'-CAGTGCCTCAACCCCATAGT-3' | 4 |
| COX-2 | sense | 5'-AGAAGGAAA TGGCTGCAGAA-3' | 5 |
| | antisense | 5'-GCTCGGCTTCCAGTATTGAG-3' | 6 |
| IL-lbeta | sense | 5'-CAGGCAGGCAGTATCACTCA-3' | 7 |
| | antisense | 5'-AGGCCACAGGTATTTTGTCG-3' | 8 |
| IL-6 | sense | 5'-GTTCTCTGGGAAATCGTGGA-3' | 9 |
| | antisense | 5'-GGAAATTGGGGTAGGAAGGA-3' | 10 |
| TNF-alpha | sense | 5'-ACGGCATGGATCTCAAAGAC-3' | 11 |
| | antisense | 5'-CGGACTCCGCAAAGTCTAAG-3' | 12 |
| GAPDH | sense | 5'-AACTTTGGCATTGTGGAAGG-3' | 13 |
| | antisense | 5'-ACACATTGGGGGTAGGAACA-3' | 14 |

At the result, it has been confirmed that the expressed level of mRNA of pro-inflammatory medicated enzyme such as iNOS or COX-2, and pro-inflammatory medicated cytokines such as IL-lbeta, IL-6, TNF-alpha in RAW264.7 cell line induced by LPS (1μg/ml) was increased in the control group treated with LPS and those in the test group pre-treated with various concentration of KS-513 compound was significantly decreased in a dose dependent manner comparing with those in the control group treated with LPS (100%), as can be seen in Table 4.

[Table 4]

| The summary for inhibitory activity on mRNA expression of pro-inflammatory cytokines by KS-513 | | | | | | | |
|---|---|---|---|---|---|---|---|
| Target gene | | Negative control | LPS treatment (1μg/ml) | KS-513 (μM) | | | |
| | | | | 2.5 | 5 | 10 | 20 |
| iNOS | Fold* | 1.00±0.10 | 8.88±2.27 | 7.25±2.5 1 | 5.94±2.9 1 | 2.48±0.1 9 | 1.32±0.4 7 |
| | IR (%) # | 88.71 | 0.06 | 18.41 | 33.09 | 72.12 | 85.19 |
| COX-2 | Fold* | 1.09±0.62 | 7.86±2.09 | 4.72±0.2 1 | 2.64±0.1 5 | 1.43±0.5 0 | 0.97±0.3 8 |
| | IR (%) # | 86.11 | 0.03 | 39.91 | 66.40 | 81.81 | 87.66 |
| TNF-al pha | Fold* | 1.00±0.08 | 11.21±0.48 | 9.60±3.3 3 | 5.34±3.2 7 | 2.72±0.9 9 | 1.49±0.7 7 |
| | IR (%) # | 91.07 | 0.03 | 14.34 | 52.39 | 75.69 | 86.70 |
| IL-lbet a | Fold* | 1.02±0.30 | 16.70±1.80 | 10.41±1. 09 | 5.72±0.2 4 | 2.56±0.1 4 | 1.70±0.5 9 |
| | IR (%) # | 93.88 | 0.01 | 37.69 | 65.75 | 84.68 | 89.85 |
| IL-6 | Fold* | 1.15±0.81 | 31.15±2.30 | 24.73±2. 26 | 14.24±1. 44 | 8.57±1.2 3 | 2.35±0.8 1 |
| | IR (%) # | 96.30 | 0.01 | 20.62 | 54.30 | 72.50 | 92.46 |
| *:value compared with a negative group#: Inhibition ratio (IR) compared with LPS treatment group | | | | | | | |

**Experimental Example 3. Inhibition effect on** iNOS protein expression and NO reproduction.

In order to determine the inhibition effect on iNOS protein expression involved in NO reproduction and the level of NO reproduction, following test was performed:

There has been known that NO shows various biological properties such as vasodilation, blood coagulation, neurotransmission, kidney function, inflammation, antitumor etc by dint of NO synthetase. NO, one of active oxygen and a highly reactive biogenic substance, plays important role in causing inflammation response and is reproduced from L-arginine by NOS (Nitric oxide synthetase). In particular, iNOS (inducible NOS) is involved in inflammatory response and expressed by the stimulation of inflammatory cytokines such as TNF-alpha, LPS (lipopolysaccharide) etc (Nathan, C., 1992, Nitric oxide as a secretory product of mammalian cells, FASEB Journal: Official publication of the Federation of American Society for Experimental Biology, 6, pp.3051-3064).

3-1. Effect on the level of iNOS expression

In order to determine the effect of test sample on the level of iNOS expression in RAW264.7 cell, following assay was performed by the method disclosed in the literature (Lee, S. U., et al., Anti-resorptive saurolactam exhibits in vitro anti-inflammatory activity via ERK-NF-kappabeta signaling pathway: International immunopharmacology, 10, pp298-303).

The expressed level of iNOS in RAW264.7 cell induced by LPS (1μg/ml) was determined by using Western blot analysis. RAW264.7 cell was inoculated into 96 well plates in the concentration of 1 x $10^6$ cell/well to incubate for 12 hours. Various concentration of KS-513 compound (2.5, 5, 10 and 20 μM) was pre-treated therewith and 1μg/mL of LPS (L6529, Sigma) was added thereto to incubate for 12 hours.

To extract protein, after washing the cell with ice-cold PBS three times, lysis buffer (50mM Tris HCl (pH 8.0), 5mM EDTA, 150 mM NaCl, 1% NP-40, 0.1% SDS, 1mM PMSF, and protease inhibitor cocktail tablet (Roche, Germany, 11697498001) was added to the cell, reacted at 4°C for 30 mins and centrifuged for 10 mins at the speed of 12,000 rpm to collect the supernatant.

The equivalent amount of the protein was isolated by SDS-PAGE and the protein was transferred to nitrocellulose membrane (BR 162-0145, Bio-Rad). The membrane was reacted with blocking buffer (1xPBS solution comprising 5% non-fat milk and 0.1% Tween 20, SH30258.01, Hyclone) for 1 hour to block non-specific binding of antibody. Antibody (Anti-iNOS) against the protein was added thereto for 1-2 hours, washed with TBST solution comprising 0.1%Tween 20 and reacted with the secondary antibody. The product was reacted with ECL system and the amount of protein was determined by imaging analysis apparatus (LAS-4000, Fujifilm).

The quantitative determination of protein was performed by determining the absorbance using assay kit (Bradford protein assay kit, Thermo) at 595nm and the result was shown in Fig. 3A.

3-2. Inhibitory effect on the NOS reproduction

In order to determine the amount of NO reproduction in medium, following test was performed according to the method disclosed in the literature (Ding, A. H., et al., 1988, Release of reactive nitrogen intermediates and reactive oxygen intermediates from mouse peritoneal macrophages, Comparison of activating cytokines and evidence for independent production, Journal of Immunology, 141, pp2407-2412).

RAW264.7 cell was inoculated into 96 well plates in the concentration of 1 x $10^5$ cell/well to incubate for 18 hours. Various concentration of KS-513 compound (2.5, 5, 10 and 20 μM) was pre-treated therewith and 1μg/mL of LPS (L6529, Sigma) was added thereto to incubate for 24 hours. The equivalent amount of Griess Reagent (G4410, Sigma) to the medium was added thereto and reacted at room temperature for 10 mins to determine the absorbance at 540nm. The level of NO in the medium was determined using by standard curve expressed according to various concentration of sodium nitrite and the result was shown in Table 5 and Fig. 3.

[Table 5]

| The summary for inhibitory activity of NO production by KS-513 | | | | |
|---|---|---|---|---|
| sample | Conc. (μM) | LPS(1 μg/mL) | NO production (relative % comparing with LPS treatment group) | Inhibitory ratio (%) |
| Negative control | 0 | - | 18.66 ± 2.01 | 81.44 |
| LPS treatment | 0 | + | 100.10 ± 3.79 | 0.00 |

(continued)

| The summary for inhibitory activity of NO production by KS-513 | | | | |
|---|---|---|---|---|
| sample | Conc. (μM) | LPS(1 μg/mL) | NO production (relative % comparing with LPS treatment group) | Inhibitory ratio (%) |
| KS-513 | 2.5 | + | 91.89 ± 4.24 | 8.21 |
| | 5 | + | 83.69 + 3.79 | 16.41 |
| | 10 | + | 80.06 ± 2.68 | 20.04 |
| | 20 | + | 72.16 ± 3.57 | 27.94 |

At the result, it has been confirmed that the expressed level of iNOS protein in the test group treated with KS-513 was reduced in a dose dependent manner while that in the LPS treatment group was increased (Fig. 3A) and the reproduced level of NO in the test group was reduced in a dose dependent manner based on that on the LPS treatment (100%).

**Experimental Example 4. Inhibition on the COX-2 protein expression and the reproduced level of PGE2.**

In order to determine the inhibitory effect on the COX-2 protein expression and the reproduced level of PGE2, following test was performed by the method disclosed in the literature (Chen, C, et al., 1999, Involvement of p38 mitogen-activated protein kinase in lipopolysaccharide-induced iNOS and COX-2 expression in J774 macrophage, Immunology, 97, pp 124-129)

The expressed level of COX-2 in RAW264.7 cell induced by LPS 1μg/ml) was determined by using Western blot analysis according to the method disclosed in Experimental Example 3.

RAW264.7 cell was inoculated into 96 well plates in the concentration of 1 x $10^5$ cell/well to incubate for 18 hours. Various concentration of KS-513 compound (2.5, 5, 10 and 20 μM) was pre-treated therewith and 1μg/mL of LPS (L6529, Sigma) was added thereto to incubate for 24 hours. The PGE2 level in the cell was determined by Enzyme immunoassay (EIA; Amersham Pharmacia) system and the result was shown in Fig. 4A, AB and Table 6.

At the result, it has been confirmed that the expressed level of COX-2 protein in the test group treated with KS-513 was reduced in a dose dependent manner while that in the LPS treatment group was increased (Fig. 4A) and the reproduced ratio of $PGE_2$ in the test group was reduced in a dose dependent manner based on that on the LPS treatment (100 %).

[Table 6]

| The summary for inhibitory activity of $PGE_2$ production | | | | |
|---|---|---|---|---|
| sample | Conc.(μM) | LPS(1 μg/mL) | $PGE_2$ production (relative % comparing with LPS treatment group) | Inhibitoryratio (%) |
| Negative control | 0 | - | 14.43 ± 0.35 | 85.57 |
| LPS treatment | 0 | + | 100.00 ± 0.92 | 0.00 |
| KS-513 | 2.5 | + | 92.68 ± 7.29 | 7.31 |
| | 5 | + | 72.87 ± 8.78 | 27.13 |
| | 10 | + | 51.65 + 8.44 | 48.35 |
| | 20 | + | 28.23 ± 4.72 | 71.77 |

**Experimental Example 5. Inhibition on the production of pro-inflammatory** cytokines.

In order to determine the inhibitory effect on the production of pro-inflammatory cytokines such as IL-lbeta, IL-6, TNF-alpha etc, following test was performed by the method disclosed in the literature (Lee, S. U., et al., Anti-resorptive saurolactam exhibits in vitro anti-inflammatory activity via ERK-NF-kappabeta signaling pathway: International immunopharmacology, 10, pp298-303).)

The inflammatory cytokines such as IL-lbeta, IL-6, TNF-alpha etc have been closely involved in the inflammation

at the various cells, for example, activated lymphocyte, macrophage etc.

RAW264.7 cell was inoculated into 96 well plates in the concentration of 1 x $10^5$ cell/well to incubate for 18 hours. Various concentration of KS-513 compound (2.5, 5, 10 and 20 μM) was pre-treated therewith and 1μg/mL of LPS (L6529, Sigma) was added thereto to incubate for 24 hours. The level of inflammatory cytokines such as IL-lbeta, IL-6, TNF-alpha etc in the cell medium was determined by ELISA kit (IL-lbeta:559603, IL-6:555240, TNF-alpha:5558534, BD) and the result was shown in Fig 5 and Table 7-9.

At the result, it has been confirmed that the level of produced pro-inflammatory cytokines such as IL-lbeta, IL-6, TNF-alpha in the test group treated with KS-513 was reduced in a dose dependent manner while that in the LPS treatment group (1μg/ml) was increased (Fig. 5). The reproduced ratio of pro-inflammatory cytokines in the test group was reduced in a dose dependent manner based on that on the LPS treatment (100 %).

[Table 7]

| The summary for inhibitory activity on cytokine production of IL-6 production | | | | |
|---|---|---|---|---|
| sample | Conc. (μM) | LPS(1 μg/mL) | IL-6 production(relative % comparing with LPS treatment group) | Inhibitory ratio (%) |
| Negative control | 0 | - | 0.00 ± 0.87 | 100 |
| LPS treatment | 0 | + | 100.00 ± 8.84 | 0.00 |
| KS-513 | 2.5 | + | 75.21 ± 2.20 | 24.79 |
| | 5 | + | 71.34 ± 5.75 | 28.66 |
| | 10 | + | 51.13 ± 2.31 | 48.87 |
| | 20 | + | 39.25 ± 2.85 | 60.75 |

[Table 8]

| sample | Conc. (μM) | LPS(1 μg/mL) | TNF-alpha production(relative % comparing with LPS treatment group) | Inhibitory ratio (%) |
|---|---|---|---|---|
| Negative control | 0 | - | 0.00 ± 1.08 | 100 |
| LPS treatment | 0 | + | 100.00 ± 5.80 | 0.00 |
| KS-513 | 2.5 | + | 97.38 ± 0.58 | 2.62 |
| | 5 | + | 86.12 ± 1.14 | 13.88 |
| | 10 | + | 68.03 ± 2.51 | 31.97 |
| | 20 | + | 58.44 ± 3.50 | 41.56 |

[Table 9]

| The summary for inhibitory activity on cytokine production of IL-lbeta production | | | | |
|---|---|---|---|---|
| sample | Conc. ( μM) | LPS(1 μg/mL) | IL-1beta production(relative% comparing with LPS treatment group) | Inhibitory ratio (%) |
| Negative control | 0 | - | 0.00 ± 0.49 | 100 |
| LPS treatment | 0 | + | 100.00 ± 4.52 | 0.00 |

(continued)

| The summary for inhibitory activity on cytokine production of IL-lbeta production | | | | |
|---|---|---|---|---|
| sample | Conc. ( μM) | LPS(1 μg/ mL) | IL-1beta production(relative% comparing with LPS treatment group) | Inhibitory ratio (%) |
| KS-513 | 2.5 | + | 95.17 ± 3.68 | 4.83 |
| | 5 | + | 76.69 ± 3.40 | 23.31 |
| | 10 | + | 61.56 ± 3.18 | 38.44 |
| | 20 | + | 46.10 ± 2.51 | 53.9 |

**Experimental Example 6. Acute toxicity test of oral administration in rat**

The acute toxicity test was performed by administrating inventive compound to 6-weeks aged SPF Sprague-Dawley rats.

250 mg/kg, 500 mg/kg, 1000 mg/kg, 5000 mg/kg of inventive compound was orally administrated to each group consisting of 2 rats and the symptoms of rats were observed for 14 days. After administrating the extract or compounds, all the clinical changes i.e., mortality, clinical signs, body weight changes was observed and blood test such as haematological test and hematological biochemistry test was performed. The abnormal changes of abdominal organ and thoracic organ were observed after autopsy.

There did not show any changes in mortality, clinical signs, body weight changes and gross findings in any group or either gender. Furthermore, there showed any toxicity in test group treated with 5000 mg/kg of inventive compound.

Accordingly, it has been confirmed that the inventive compound prepared in the present invention was potent and safe substance showing $LD_{50}$ (more than 5000 mg/kg) in oral administration.

**Mode for the Invention**

Hereinafter, the formulating methods and kinds of excipients will be described, but the present invention is not limited to them. The representative preparation examples were described as follows.

[Table 10]

| Preparation of injection | |
|---|---|
| KS 513 | 100 mg |
| Sodium metabisulfite | 3.0 mg |
| Methyl paraben | 0.8 mg |
| Propyl paraben | 0.1 mg |
| Distilled water for injection | optimum amount |

Injection preparation was prepared by dissolving active component, controlling pH to about 7.5 and then filling all the components in 2 mL ample and sterilizing by conventional injection preparation method.

[Table 11]

| Preparation of powder | |
|---|---|
| KS 513 | 500 mg |
| Corn Starch | 100 mg |
| Lactose | 100 mg |
| Talc | 10 mg |

Powder preparation was prepared by mixing above components and filling sealed package.

[Table 12]

| Preparation of tablet | |
|---|---|
| KS 513 | 200 mg |
| Corn Starch | 100 mg |
| Lactose | 100 mg |
| Magnesium stearate | optimum amount |

Tablet preparation was prepared by mixing above components and entabletting.

[Table 13]

| Preparation of capsule | |
|---|---|
| KS 513 | 100 mg |
| Lactose | 50 mg |
| Corn starch | 50 mg |
| Talc | 2 mg |
| Magnesium stearate | optimum amount |

Tablet preparation was prepared by mixing above components and filling gelatin capsule by conventional gelatin preparation method.

[Table 14]

| Preparation of liquid | |
|---|---|
| KS 513 | 1000 mg |
| Sugar | 20 g |
| Polysaccharide | 20 g |
| Lemon flavor | 20 g |

Liquid preparation was prepared by dissolving active component, and then filling all the components in 1000 mL ample and sterilizing by conventional liquid preparation method

[Table 15]

| Preparation of health food | |
|---|---|
| KS 513 | 1000 mg |
| Vitamin mixture | optimum amount |
| Vitamin A acetate | 70 g |
| Vitamin E | 1.0 mg |
| Vitamin B10 | 13mg |
| Vitamin B2 | 0.15mg |
| Vitamin B6 | 0.5 mg |
| Vitamin B1 | 20.2g |
| Vitamin C | 10 mg |
| Biotin | 10g |
| Amide nicotinic acid | 1.7 mg |

(continued)

| Preparation of health food | |
|---|---|
| Folic acid | 50 g |
| Calcium pantothenic acid | 0.5 mg |
| Mineral mixture | optimum amount |
| Ferrous sulfate | 1.75 mg |
| Zinc oxide | 0.82 mg |
| Magnesium carbonate | 25.3 mg |
| Monopotassium phosphate | 15 mg |
| Dicalcium phosphate | 55 mg |
| Potassium citrate | 90 mg |
| Calcium carbonate | 100 mg |
| Magnesium chloride | 24.8 mg |

The above mentioned vitamin and mineral mixture may be varied in many ways. Such variations are not to be regarded as a departure from the scope of the present invention as defined by the appended claims.

[Table 16]

| Preparation of health beverage | |
|---|---|
| KS 513 | 1000 mg |
| Citric acid | 1000 mg |
| Oligosaccharide | 100 g |
| Apricot concentration | 2 g |
| Taurine | 1 g |
| Distilled water | 900 mL |

Health beverage preparation was prepared by dissolving active component, mixing, stirred at 85°C for 1 hour, filtered and then filling all the components in 1000 mL ample and sterilizing by conventional health beverage preparation method.

The invention being thus described, it will be obvious that the same may be varied in many ways. Such variations are not to be regarded as a departure from the scope of the present invention, and all such modifications as would be obvious to one skilled in the art are intended to be included within the scope of the invention as defined by the appended claims.

**Industrial Applicability**

As described in the present invention, inventive KS513 compound from the extract of *Pseudolysimachion* rotundum var subintegrum showed potent anti-inflammatory, anti-allergy, anti-asthma and anti-COPD activity confirmed by through various tests, for example, (1) cyto-toxicity test using by RAW264.7 cell line, (2) an effect on the mRNA expression of pro-inflammatory medicated enzyme such as iNOS or COX-2, and pro-inflammatory medicated cytokines such as IL-lbeta, IL-6, TNF-alpha in RAW264.7 cell line induced by LPS, (3) an inhibition effect on iNOS protein expression involved in NO reproduction and the level of NO reproduction, (4) an inhibition effect on the COX-2 protein expression and the reproduced level of PGE2, (5) an inhibition effect on the produce level of IL-1beta, IL-6, TNF-alpha etc. Therefore, it can be used as the therapeutics or functional health food for treating and preventing allergic disease, inflammatory disease, asthma or chronic obstructive pulmonary disease (COPD).

## Claims

1. (1aS,1bS,2S,4S,5aR,6S,6aS)-1a-(hydroxymethyl)-4-methoxy-2-(((2S,3R,4S,5S,6R)-3,4,5-trihydroxy-6-(hydroxymethyl)tetrahydro-2H-pyran-2-yl)oxy)octahydrooxireno[2',3':4,5]cyclopenta[1,2-c]pyran-6-yl4-hydroxybenzoate (KS-513) represented by the following chemical formula (1), a pharmaceutically acceptable salt or solvates thereof:

[Chemical Formula 1]

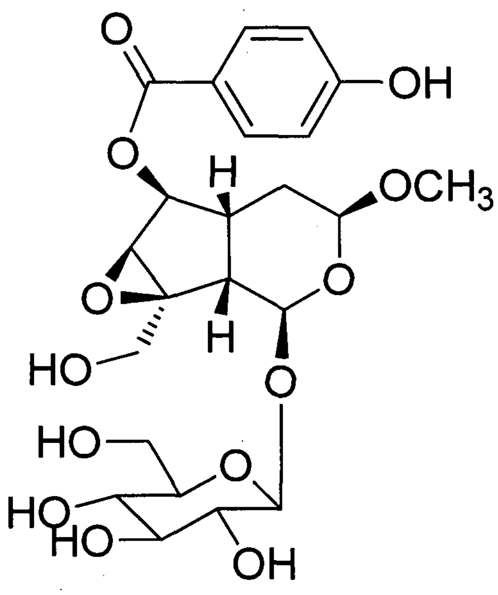

(1).

2. A pharmaceutical composition comprising the compound (KS513), the pharmaceutically acceptable salt or solvates thereof as set forth in claim 1.

3. The pharmaceutical composition of claim 2 further comprising a pharmaceutically acceptable carrier or excipient.

4. The compound (KS513), or the pharmaceutically acceptable salt or solvates thereof as set forth in claim 1, or the pharmaceutical composition as set forth in claims 2 or 3 for use in a method of treating or preventing allergic disease, inflammatory disease, asthma or chronic obstructive pulmonary disease (COPD) in mammals, wherein the method comprises administering a therapeutically effective amount of said compound (KS513), said pharmaceutically acceptable salt or solvates thereof or of said pharmaceutical composition into the mammal suffering from allergic disease, inflammatory disease, asthma or chronic obstructive pulmonary disease (COPD).

5. A health functional food comprising a therapeutically effective amount of the compound (KS513), the pharmaceutically acceptable salt or solvates thereof as set forth in claim 1 as an active ingredient.

6. A health care food comprising a therapeutically effective amount of the compound (KS513), the pharmaceutically

acceptable salt or solvates thereof as set forth in claim 1, together with a sitologically acceptable additive.

**7.** The health functional food of claim 5 for use in a method of preventing or alleviating allergic disease, inflammatory disease, asthma or chronic obstructive pulmonary disease (COPD).

**8.** The health care food of claim 6 for use in a method of preventing or alleviating allergic disease, inflammatory disease, asthma or chronic obstructive pulmonary disease (COPD).

## Patentansprüche

**1.** (1aS,1bS,2S,4S,5aR,6S,6aS)-1a-(Hydroxymethyl)-4-methoxy-2-(((2S,3R,4S, 5S,6R)-3,4,5-trihydroxy-6-(hydroxymethyl)tetrahydro-2H-pyran-2-yl)oxy)octahydrooxireno[2',3':4,5]cyclopenta[1,2-c]pyran-6-yl4-hydroxybenzoat (KS-513), dargestellt durch die folgende chemische Formel (1), ein pharmazeutisch annehmbares Salz oder Solvate davon:

[Chemische Formel 1]

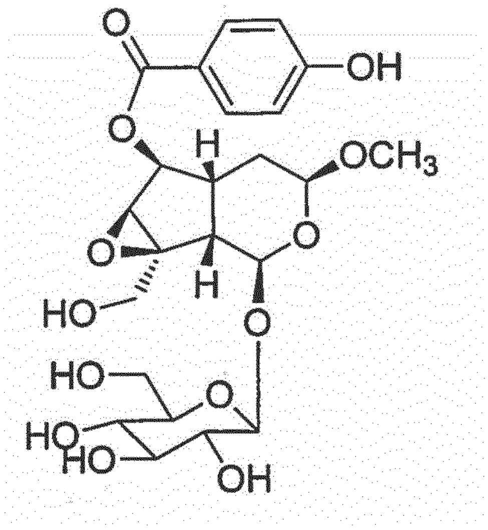

(1).

**2.** Pharmazeutische Zusammensetzung, umfassend die Verbindung (KS513), das pharmazeutisch annehmbare Salz oder Solvate davon, wie in Anspruch 1 dargelegt.

**3.** Pharmazeutische Zusammensetzung nach Anspruch 2, weiterhin einen pharmazeutisch annehmbaren Träger oder Hilfsstoff umfassend.

**4.** Verbindung (KS513) oder das pharmazeutisch annehmbare Salz oder Solvate davon, wie in Anspruch 1 dargelegt, oder die pharmazeutische Zusammensetzung, wie in Anspruch 2 oder 3 dargelegt, zur Verwendung in einem Verfahren zum Behandeln oder Verhindern von allergischer Krankheit, entzündlicher Erkrankung, Asthma oder chronisch obstruktiver Lungenerkrankung (COPD) bei Säugern, wobei das Verfahren die Verabreichung einer therapeutisch wirksamen Menge der Verbindung (KS513), des pharmazeutisch annehmbaren Salzes oder der Solvate davon oder der pharmazeutischen Zusammensetzung an den an allergischer Krankheit, entzündlicher Erkrankung, Asthma oder chronisch obstruktiver Lungenerkrankung (COPD) leidenden Säuger umfasst.

**5.** Funktionelles Gesundheitsnahrungsmittel, umfassend eine therapeutisch wirksame Menge der Verbindung (KS513), des pharmazeutisch annehmbaren Salzes oder der Solvate davon, wie in Anspruch 1 dargelegt, als Wirkstoff.

**6.** Gesundheitsförderndes Nahrungsmittel, umfassend eine therapeutisch wirksame Menge der Verbindung (KS513), des pharmazeutisch annehmbaren Salzes oder der Solvate davon nach Anspruch 1, zusammen mit einem nahrungsmittelkundlich annehmbaren Zusatzstoff.

7. Funktionelles Gesundheitsnahrungsmittel nach Anspruch 5 zur Verwendung in einem Verfahren zum Vorbeugen oder Lindern einer allergischen Erkrankung, einer entzündlichen Erkrankung, Asthma oder einer chronisch obstruktiven Lungenerkrankung (COPD).

8. Gesundheitsförderndes Nahrungsmittel nach Anspruch 6 zur Verwendung in einem Verfahren zum Vorbeugen oder Lindern einer allergischen Erkrankung, einer entzündlichen Erkrankung, von Asthma oder einer chronisch obstruktiven Lungenerkrankung (COPD).

**Revendications**

1. 4-Hydroxybenzoate de (1aS,1bS,2S,4S,5aR,6S,6aS)-1a-(hydroxyméthyl)-4-méthoxy-2-(((2S,3R,4S,5S,6R)-3,4,5-trihydroxy-6-(hydroxyméthyl)tétrahydro-2H-pyran-2-yl)oxy)octahydrooxyréno[2',3':4,5]cyclopenta[1,2c]pyran-6-yle (KS513) représenté par la formule chimique (1) suivante, sel ou solvates pharmaceutiquement acceptables de celui-ci :

[Formule chimique 1]

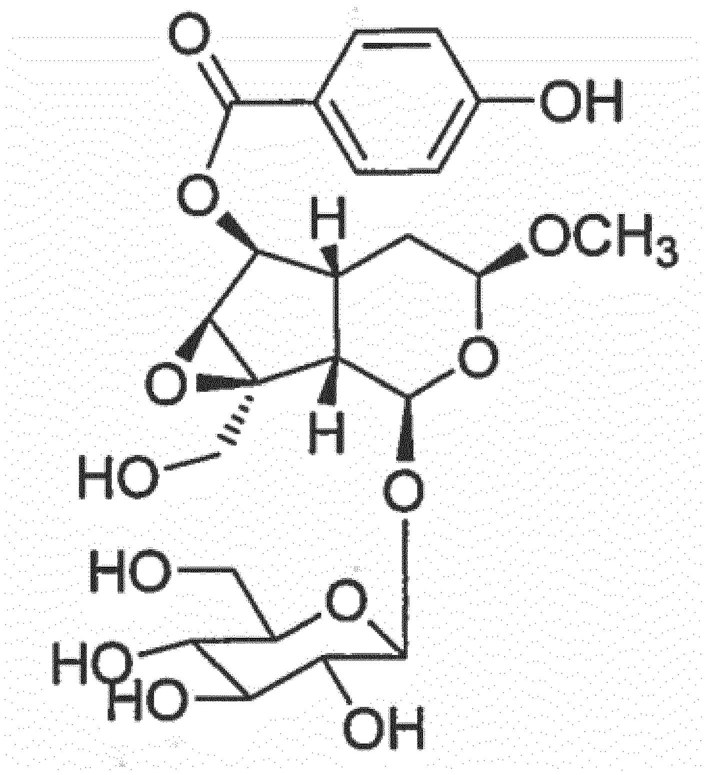

(1).

2. Composition pharmaceutique comprenant le composé (KS513), le sel ou les solvates pharmaceutiquement acceptables de celui-ci ainsi que présentés dans la revendication 1.

3. Composition pharmaceutique selon la revendication 2 comprenant en outre un véhicule ou excipient pharmaceutiquement acceptable.

4. Composé (KS513), ou sel ou solvates pharmaceutiquement acceptables de celui-ci ainsi que présentés dans la revendication 1, ou composition pharmaceutique ainsi que présentée dans les revendications 2 ou 3 destinés à être

utilisés dans un procédé de traitement ou de prévention d'une maladie allergique, d'une maladie inflammatoire, de l'asthme ou d'une bronchopneumopathie chronique obstructive (BPCO) chez des mammifères, le procédé comprenant l'administration d'une quantité efficace sur le plan thérapeutique dudit composé (KS513), dudit sel ou desdits solvates pharmaceutiquement acceptables de celui-ci au mammifère souffrant de maladie allergique, de maladie inflammatoire, d'asthme ou de bronchopneumopathie chronique obstructive (BPCO).

**5.** Aliment à fonction de santé comprenant une quantité efficace sur le plan thérapeutique du composé (KS513), du sel ou des solvates pharmaceutiquement acceptables de celui-ci ainsi que présentés dans la revendication 1 en tant que principe actif.

**6.** Aliment à fonction de santé comprenant une quantité efficace sur le plan thérapeutique du composé (KS513), du sel ou des solvates de celui-ci ainsi que présentés dans la revendication 1, associés à un additif acceptable sur le plan sitologique.

**7.** Aliment à fonction de santé selon la revendication 5 destiné à être utilisé dans un procédé pour la prévention ou le soulagement d'une maladie allergique, d'une maladie inflammatoire, de l'asthme ou d'une bronchopneumopathie chronique obstructive (BPCO).

**8.** Aliment à fonction de santé selon la revendication 6 destiné à être utilisé dans un procédé pour la prévention ou le soulagement d'une maladie allergique, d'une maladie inflammatoire, de l'asthme ou d'une bronchopneumopathie chronique obstructive (BPCO).

[Fig. 1]

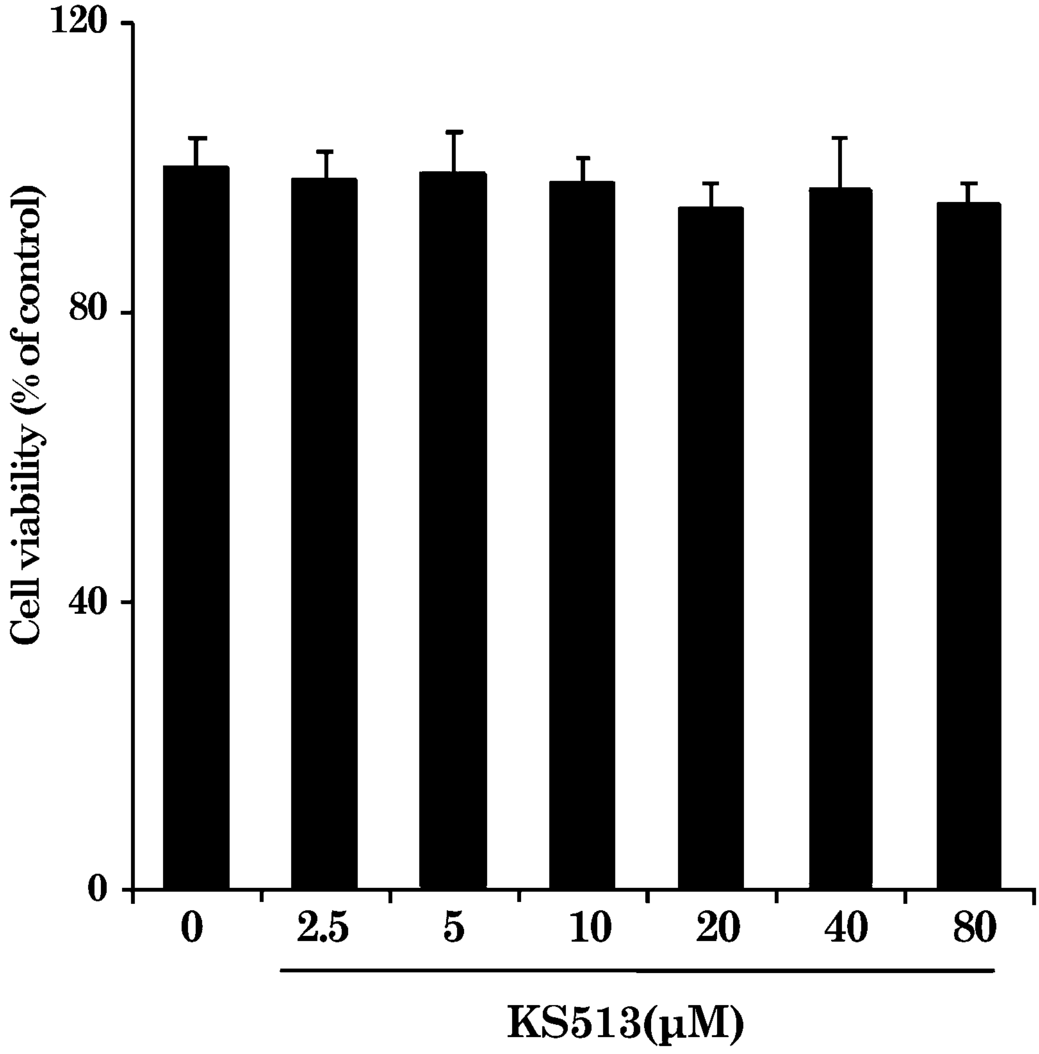

[Fig. 2]

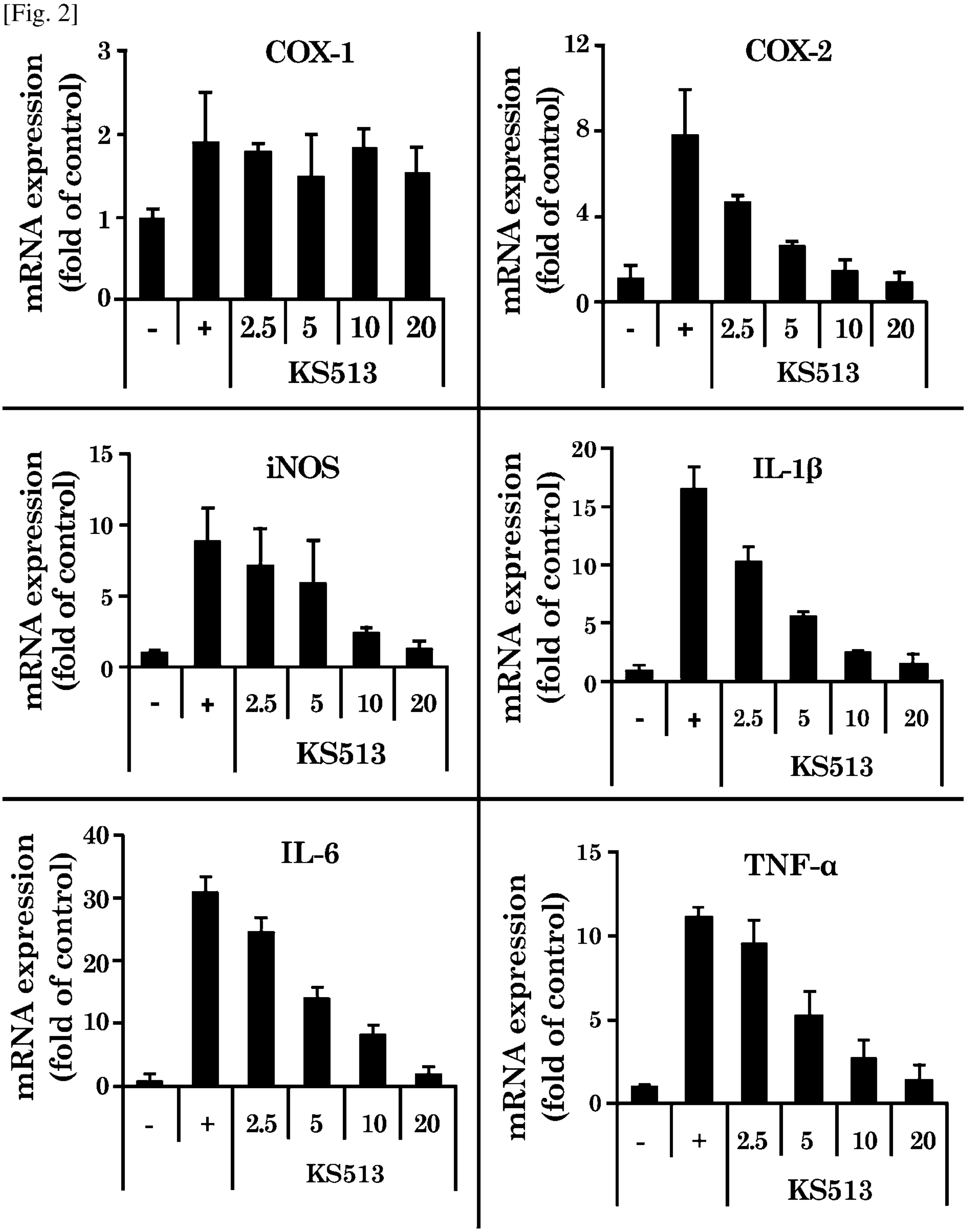
COX-1
COX-2
iNOS
IL-1β
IL-6
TNF-α
mRNA expression (fold of control)
- + 2.5 5 10 20
KS513

[Fig. 3]

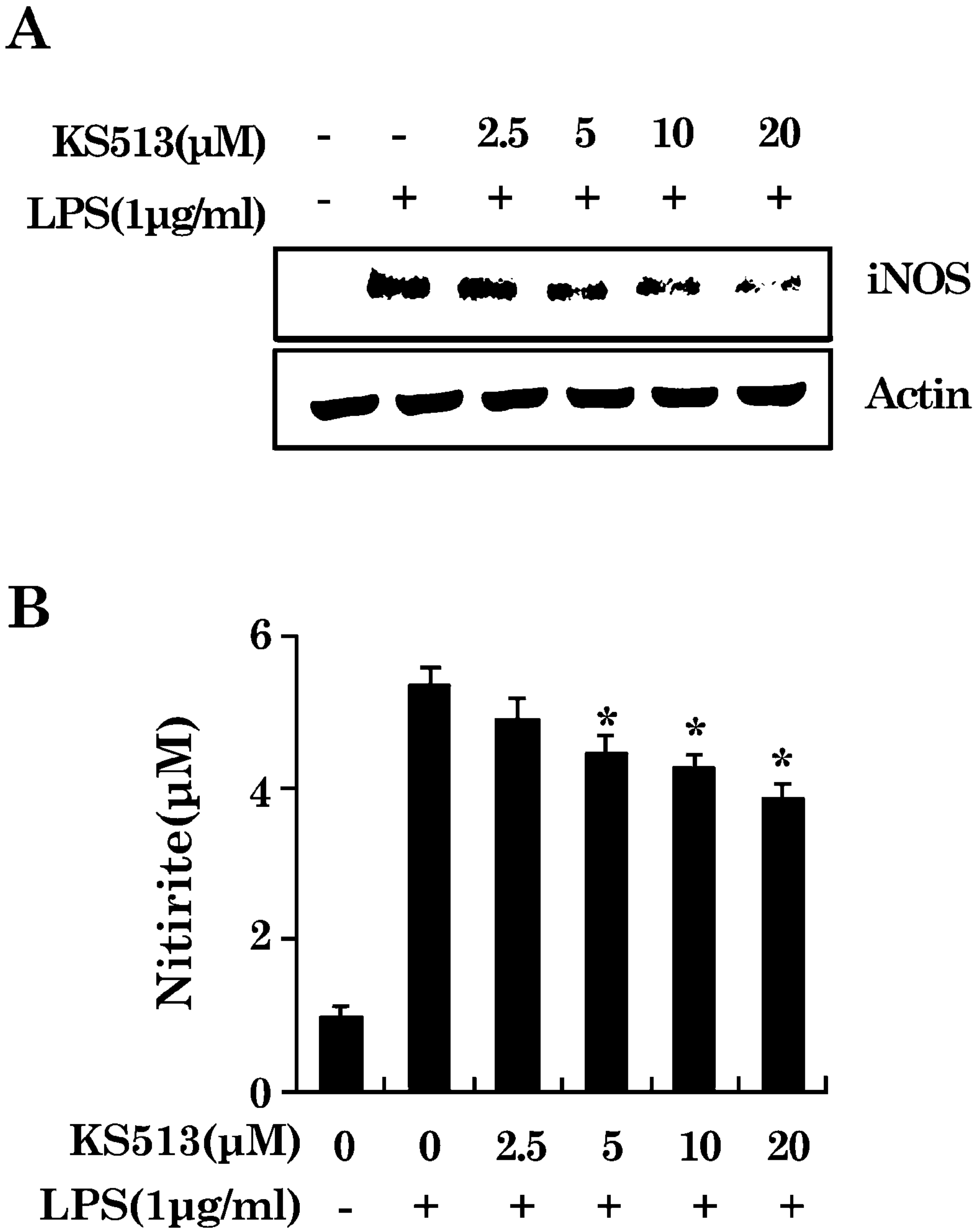
A
KS513(μM) - - 2.5 5 10 20
LPS(1μg/ml) - + + + + +
iNOS
Actin
B
Nitirite(μM)
0
2
4
6
*
*
*
KS513(μM) 0 0 2.5 5 10 20
LPS(1μg/ml) - + + + + +

[Fig. 4]

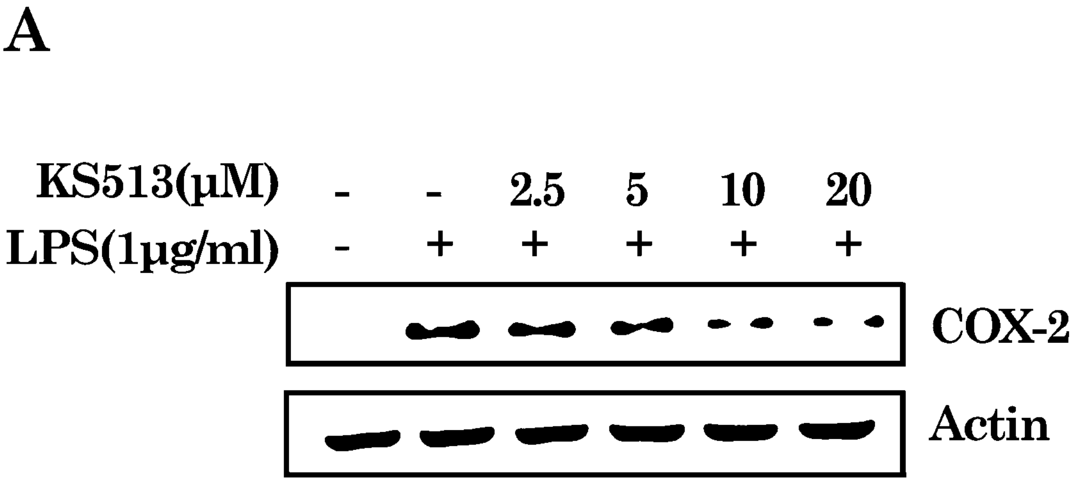
A
KS513(μM) - - 2.5 5 10 20
LPS(1μg/ml) - + + + + +
COX-2
Actin

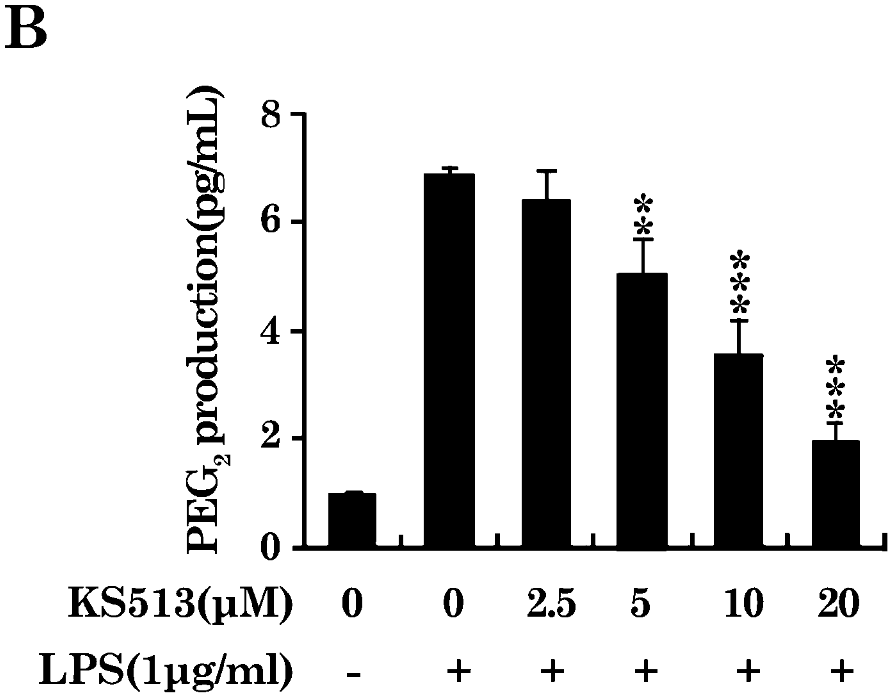
B
PEG2 production(pg/mL)
8
6
4
2
0
**
***
***
KS513(μM) 0 0 2.5 5 10 20
LPS(1μg/ml) - + + + + +

[Fig. 5]

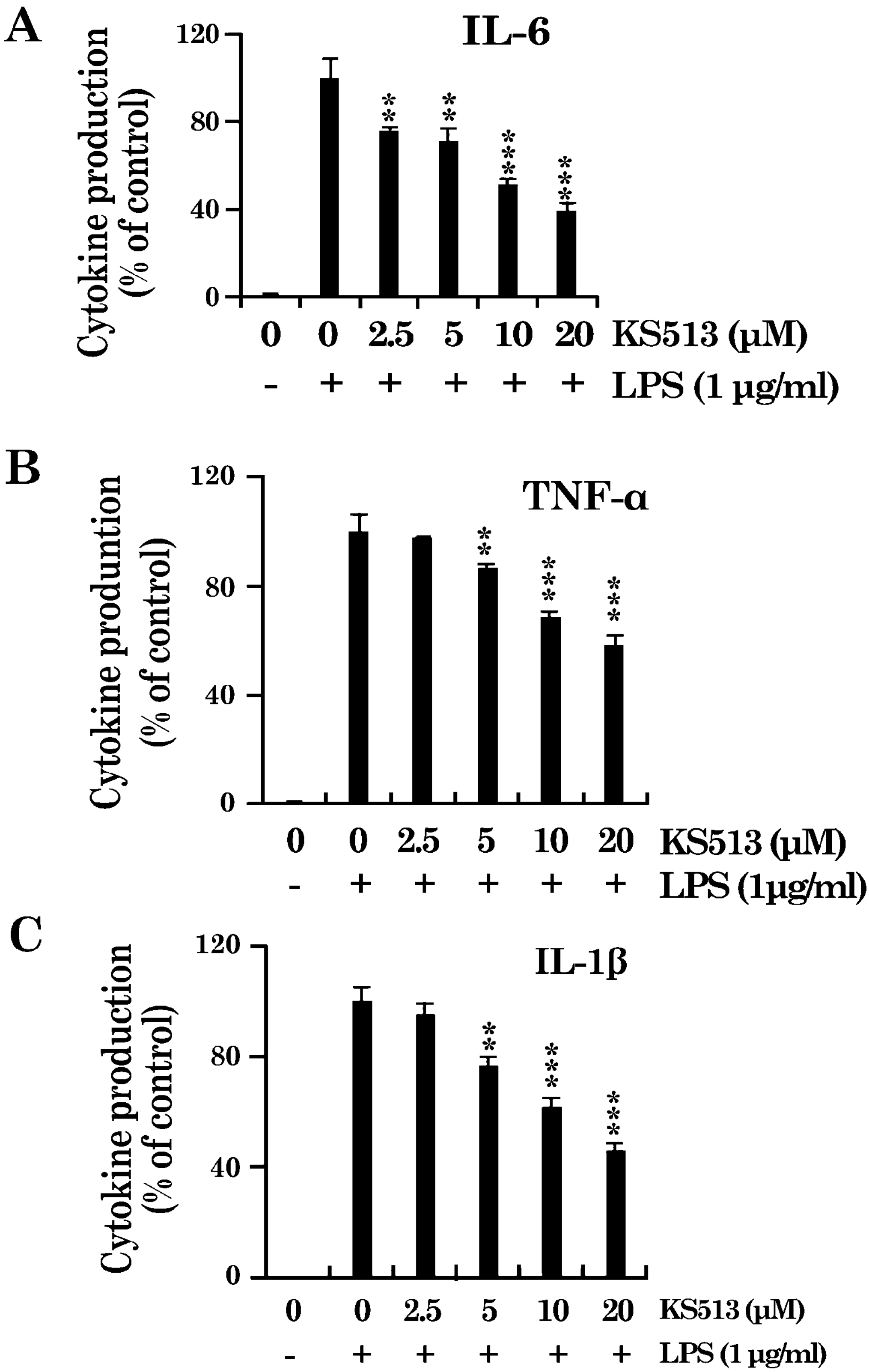
A
IL-6
Cytokine production
(% of control)
120
80
40
0
0 0 2.5 5 10 20 KS513 (μM)
- + + + + + LPS (1 μg/ml)
B
TNF-α
Cytokine prodution
(% of control)
120
80
40
0
0 0 2.5 5 10 20 KS513 (μM)
- + + + + + LPS (1μg/ml)
C
IL-1β
Cytokine production
(% of control)
120
80
40
0
0 0 2.5 5 10 20 KS513 (μM)
- + + + + + LPS (1 μg/ml)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 10860080 B1
- US 20120183632 A1
- KR 102006125499 A1
- KR 1476045 B1
- KR 1504651 B1
- WO 2011101672 A1
- KR 1476095 B1
- US 2009324750 A
- KR 102006125499
- WO 1020150085752 A


### Non-patent literature cited in the description


- Pathophysiology of asthma. **MINOGUCHI K ; ADACHI M.** Rehabilitation of the patient with respiratory disease. McGraw-Hill, 1999, 97-104
- **ELIAS JA et al.** *J Clin Invest.,* 2003, vol. 111, 291-297
- **MAGGI E. et al.** *Immunotechnology,* 1998, vol. 3, 233-244
- **PAWANKAR R. et al.** *Curr. Opin. Allergy Clin. Immunol.,* 2001, vol. 1, 3-6
- **BARNES PJ et al.** *Phamacol. Rev.,* 1998, vol. 50, 515-596
- **BARNES P.J.** *Pharmacol. Rev.,* 2004, vol. 56, 515-548
- **KIM, D. S. et al.** *Am. J. Respir. Crit. Care Med.,* 2005, vol. 172, 842-847
- **SIN, D.D. et al.** *Proc. Am. Thorac. Soc.,* 2005, vol. 2, 8-11
- **BUIST A.S. et al.** *Lancet,* 2007, vol. 370, 741-750
- **BARNES PJ.** *Chest,* 2000, vol. 117, 10S-14S
- **SAETTA M. et al.** *Am. J. Respir. Crit. Care Med.,* 2001, vol. 163, 1304-1309
- **SAETTA M. et al.** *Am. J. Respir. Crit. Care Med.,* 1998, vol. 157, 822-826
- **DI STEFANO A. et al.** *Am. J. Respir. Crit. Care Med.,* 1998, vol. 158, 1277-1285
- **FABBRI L. et al.** *Thorax,* 1998, vol. 53, 803-808
- **FABBRI LM. et al.** *Am. J. Respir. Crit. Care Med.,* 2003, vol. 167, 418-424
- **HELE D. et al.** *Expert. Opino. Invest. Drug,* 2003, vol. 12, 5-18
- **FOX, J C. et al.** *Curr. Opin. Pharmacol.,* 2009, vol. 9, 231-242
- **SONG, H. H. et al.** Piscroside C, a novel iridoid glycoside isolated from Pseudolysimachion rotundum var. subinegrum suppresses airway inflammation induced by cigarette smoke. *Journal of Ethnopharmacology,* vol. 170, 20-27
- **TASKOVA R. et al.** Iridoid glucosides of the genus Veronica s.l. and their systematic significance. *Plant Systematics and Evolution,* vol. 231 (1-4), 1-17
- **JIA, Q. et al.** Pikuroside: a novel iridoid from Picrorhiza kurroa. *Journal of Natural Products,* vol. 62 (6), 901-903
- **ROWE, RAYMOND C et al.** Handbook of Pharmaceutical Excipients. Pharmaceutical Press, 2012
- *Codex General Standard for Food Additives, www.codexalimentarius.net/gsfaonline/index.html*
- **LEE, S. U. et al.** Anti-resorptive saurolactam exhibits in vitro anti-inflammatory activity via ERK-NK-kappa B signaling pathway. *International Immunopharmacology,* 2010, vol. 10, 298-303
- **LIVAK, K.J. ; SCHIMITTGEN T.D.** Analysis of relative gene expression data using real-time quantitiative PCR and the 2 (-Delta Delta C(T) method. *Methods,* 2001, vol. 25, 402-408
- **NATHAN, C.** Nitric oxide as a secretory product of mammalian cells. *FASEB Journal: Official publication of the Federation of American Society for Experimental Biology,* 1992, vol. 6, 3051-3064
- **LEE, S. U. et al.** Anti-resorptive saurolactam exhibits in vitro anti-inflammatory activity via ERK-NF-kappabeta signaling pathway. *International immunopharmacology,* vol. 10, 298-303
- **DING, A. H. et al.** Release of reactive nitrogen intermediates and reactive oxygen intermediates from mouse peritoneal macrophages, Comparison of activating cytokines and evidence for independent production. *Journal of Immunology,* 1988, vol. 141, 2407-2412
- **CHEN, C et al.** Involvement of p38 mitogen-activated protein kinase in lipopolysaccharide-induced iNOS and COX-2 expression in J774 macrophage. *Immunology,* 1999, vol. 97, 124-129

<110> YUNGJIN PHARMACEUTICAL CO., LTD. Korea Research Institute of Bioscience and Biotechnology

<120> A NOVEL COMPOUND (KS 513) ISOLATED FROM PSEUDOLYSIMACHION ROTUNDUM VAR. SUBINTEGRUM, THE COMPOSITION COMPRISING THE SAME AS AN ACTIVE INGREDIENT FOR PREVENTING OR TREATING ALLERGY DISEASE, INFLAMMATORY DISEASE, ASTHMA OR CHRONIC OBSTRUCTIVE PULMONARY DISEASE AND THE USE THEREOF

<130> YGC150177MR

<150> 10-2015-0085752
<151> 2015-06-17

<160> 14

<170> KoPatentIn

<210> 1
<211> 20
<212> DNA
<213> Homo Sapiens

<400> 1
ccttgttcag ctacgccttc 20

<210> 2
<211> 20
<212> DNA
<213> Homo Sapiens

<400> 2
aaggccaaac acagcatacc 20

<210> 3
<211> 20
<212> DNA
<213> Homo Sapiens

<400> 3
gtggctattt cctgcagctc 20

<210> 4
<211> 20
<212> DNA
<213> Homo Sapiens

<400> 4
cagtgcctca accccatagt 20

<210> 5
<211> 20
<212> DNA
<213> Homo Sapiens

<400> 5
agaaggaaat ggctgcagaa 20

<210> 6
<211> 20
<212> DNA
<213> Homo Sapiens

<400> 6
gctcggcttc cagtattgag 20

<210> 7
<211> 20
<212> DNA
<213> Homo Sapiens

<400> 7
caggcaggca gtatcactca 20

<210> 8
<211> 20
<212> DNA
<213> Homo Sapiens

<400> 8
aggccacagg tattttgtcg 20

<210> 9
<211> 20
<212> DNA
<213> Homo Sapiens

<400> 9
gttctctggg aaatcgtgga 20

<210> 10
<211> 20
<212> DNA
<213> Homo Sapiens

<400> 10
ggaaattggg gtaggaagga 20

<210> 11
<211> 20
<212> DNA
<213> Homo Sapiens

<400> 11
acggcatgga tctcaaagac 20

<210> 12
<211> 20
<212> DNA
<213> Homo Sapiens

<400> 12
cggactccgc aaagtctaag 20

<210> 13
<211> 20
<212> DNA
<213> Homo Sapiens

<400> 13
aactttggca ttgtggaagg 20

<210> 14
<211> 20
<212> DNA
<213> Homo Sapiens

<400> 14
acacattggg ggtaggaaca 20